Europäisches Patentamt

(19) European Patent Office

Office européen des brevets

(11) EP 0 673 652 B1

(12) EUROPEAN PATENT SPECIFICATION

(45) Date of publication and mention
of the grant of the patent:
13.03.2002 Bulletin 2002/11

(51) Int Cl.$^7$: **A61K 35/48**, A61K 35/12, C07K 2/00

(21) Application number: 94905611.3

(22) Date of filing: 20.07.1993

(86) International application number:
PCT/UA93/00003

(87) International publication number:
WO 94/02104 (03.02.1994 Gazette 1994/04)

(54) **BIOLOGICALLY ACTIVE AGENT HAVING IMMUNOMODULATING PROPERTIES, METHOD FOR ITS OBTAINING AND PHARMACEUTICAL PREPARATION BASED ON IT**

BIOLOGISCH AKTIVE SUBSTANZ MIT IMMUNMODULIERENDEN EIGENSCHAFTEN, VERFAHREN ZU IHRER GEWINNUNG UND AUS IHR HERGESTELLTE, PHARMAZEUTISCHE ZUBEREITUNG

AGENT BIOLOGIQUEMENT ACTIF AYANT DES PROPRIETES D'IMMUNOMODULATION, PROCEDE POUR SA PRODUCTION ET PREPARATION PHARMACEUTIQUE A BASE DE CET AGENT

(84) Designated Contracting States:
AT BE CH DE FR GB GR LI NL

(30) Priority: 21.07.1992 RU 5063049
17.12.1992 UA 93030288

(43) Date of publication of application:
27.09.1995 Bulletin 1995/39

(73) Proprietor: NIKOLAENKO; Alexandr, Nikolaevich
Kiev, 252049 (UA)

(72) Inventors:
• NIKOLAENKO, Alexandr Nikolaevich
Kiev, 252049 (UA)

• SHOROKH, Dmitriy Borislavovich
Kiev, 252192 (UA)
• SHEVCHENKO, Alexandr Alexandrovich
Kiev, 252156 (UA)

(74) Representative:
von Füner, Alexander, Prof.h.c. Dr.
v. Füner Ebbinghaus Finck Hano
Mariahilfplatz 2 & 3
81541 München (DE)

(56) References cited:
EP-A- 0 083 673          EP-A- 0 109 089
EP-A- 0 249 563          EP-A- 0 285 370
FR-A- 2 272 683          FR-A- 2 352 549
FR-A- 2 469 926          FR-A- 2 599 972

**Description**

FIELD OF THE INVENTION

The group of inventions belongs to the chemical - pharmaceutical industry, namely to the field of producing biologically active substances with immunomodulating action for veterinary and medical usage aimed at the normalization of physiological state in human and animal organisms, including an improvement of animal resistance and productivity.

In accordance with this invention, a biologically active substance is referred to substances of animal origin and includes organic compounds.

Various pathological states in animal and human organisms cause parameters of their physiological balance to deviate from the norm. In these cases, curative influence on the organism, including prophylaxis, means stabilization, that is maintenance of physiological parameters in the norm.

The group of inventions deals with a biologically active substance, a method of its production, a preparation based on it, and a method for the normalization of physiological state of human and animal organisms using the preparation.

BACKGROUND OF THE INVENTION

The normalization of physiological state of human and animals can be achieved in two directions: firstly, by means of direct influence on pathology and, secondly, by means of indirect influence on systems, organs and tissues of the organism, which are responsible for the parameters of homeostasis to be maintained in the norm.

The administration of hormonal preparations, mediators, growth factors, chemotherapeutic drugs and similar preparations provides direct influence on target cells by means of reception. For example, in the case of treatment of diabetes mellitus, administered insulin, compensates functional insufficiency of the pancreas. In the case of disorders in functional activity of the nervous system, neuromediators are to be administered because of their ability to normalize the threshold sensitivity of neurones, and consequently improve the transmission of neural impulses. In the case of disorders in functional activity of the immune system, immunomediators able to correct immune response should be used, etc.

In the case of mediated influence on the organism, the treatment of diseases is achieved by means of mobilization of organism internal reserves. For instance, in the processes connected with regeneration of tissues and organs, their rehabilitation and recovery of their functions, as well as in the cases of inflammatory and infectious processes, it is necessary to stimulate the immune system of organism. Owing to this stimulation, an improvement in immunological as well as general state of the organism may be achieved.

There is a known biologically active substance on the basis of embryonic tissue, with immunomodulating activity, able to restore physiological state of the organism and regulate cellular metabolism (FR-A-2413912). This biologically active substance (herein after BAS) is prepared on the basis of ingredients of disintegrated and homogenized animal embryonic tissues in a mixture with a physiological solution.

Due to the presence of an immunomodulating factor in its composition, a BAS has a comparatively high immunomodulating activity, thereby ensuring the normalization of physiological state of the organism, and resulting in an improvement of animal productivity by 5-15% in comparison with control.

However, an effectiveness of this known BAS is insufficient so far as it includes, alongside of immunomodulating factors, also immunodepressants which prevent full realization of its immunomodulating effect. As a matter of fact, the immunomodulating activity of this BAS is an integral effect ensured by the presence of immunostimulating factors and depressants at the same time. Thus, the actual specific activity of this known BAS, which depends upon a ratio between immunostimulating factors and depressants, is relatively low, as a consequence of a high concentration of depressants.

Another disadvantage of this known BAS is its high content of high-molecular compounds. These compounds are immunogenes that cause various side immune reactions (allergy, delayed hypersensitivity and so on).

To a considerable extent, all disadvantages of the described BAS depend upon a method of its production, where the technological process finishes with the operation of disintegration and homogenization of raw material from animal tissue, which results in the inevitable presence of a significant quantity of depressants and high-molecular compounds in its composition.

There is also well known BAS with immunomodulating activity for the normalization of physiological state of the organism. These BASes are prepared on the basis of disintegrated and homogenized tissue, extracted from animal organs in the form:

- of embryonic tissue extract (EP-A-0249563);

- or bone marrow tissue extract in a mixture with a physiological solution which allows the engraftment of allogenic bone marrow and induces persistent chimerism in previously lethally irradiated animals, as well as survival of the latter (FR-A-2469926).

The process of its production comprises disintegration and homogenization of raw material from animal tissue, with subsequent removing insoluble compounds and using extracted soluble substances as desired product (EP-A-0249563).

The specific activity of substances, obtained by the described method, and determined by resulting correlation between the effects of immunostimulation and immunoinhibition, is also insufficient. It could be explained by the fact, that in the process of obtaining a well known BAS in the form of extract only soluble proteins were used, and structural proteins were removed, that significantly reduced the content of immunostimulating fators in this substance composition.

Besides, the well known BAS also comprises some high-molecular compounds causing side immune reactions of the organism.

There is also a known BAS on the basis of disintegrated and homogenized animal tissue, which was obtained as a extract from thermostable components of tissue of the immunocompetent organs (thymus, spleen, lymphatic nodules) or hormone producing tissues (placenta) for the normalization of physiological state of the organism including an improvement of agricultural animal resistance and productivity (SU-A-1442064).

The well known BAS is designed for the regulation of immune T-system, and practically it does not cause any side immune reactions (allergies, delayed hypersensitivity and so on) in the case of its usage. Its specific activity, in comparison with mentioned BASes, is higher because of comparatively higher content of immunostimulating factors.

However using of this substance for the normalization of physiological state of the organism is limited, because of its mediator and/or hormonal nature, which does not allow overdosages related with a danger that the parameters of organism homeostasis could be disordered.

For the normalization of physiological state of the organism, the usage of a BAS obtained as an extract from thermostable components of disintegrated animal tissue, where mediator and/or hormone producing activity is not so significant, is more safe (SU-A-139404). This BAS has same qualities, as the described BASes, but without mediator and/or hormonal activity, despite a decrease in its immunomodulating activity. These properties of the well known BAS are linked with the method of its production, comprising disintegration of animal tissue, its twofold thermoprocessing and packaging under aseptic conditions (SU-A-139404).

The other BAS having immunomodulating properties, for the normalization of physiological state of the organism, including healing of damaged parts of the body, is prepared on the basis of the water-soluble hydrolysis products of raw material from disintegrated animal tissue, taken preferably from immature animals (US-A-4455302). Besides, it includes some mineral substances, soluble organic compounds, such as polypeptides, amino acids and mineral substances, obtained as a result of tissue hydrolysis in its content .

This substance has high effectiveness in normalizing the parameters of physiological state of an organism. It could be explained by the fact, that a well known BAS is characterized by the high correlation between the effects of immunomstimulation and immunoinhibition due to more higher content of some immunostimulating factors in its content.

But an effectiveness of the described BAS is insufficient because of a great amount of ballast substances and immunodepressants in its content. Besides, the well known BAS also may cause some side effects, noted during its use (allergy and others). A disadvantage of the known BAS is also the fact that composition of desired product is not permanent and fluctuates from one batch to another, that causes the difficulties in its standardization.

The properties of a well known BAS, and first of all its immunomodulating activity, are directly connected with the process of its production.

The well known method of processing a BAS having immunomodulating activity for the normalization of physiological state of the organism includes hydrolysis of the washed and disintegrated raw material from animal tissue with subsequent centrifugation, filtration of the hydrolysis product and extraction of desired product in the form of powder or gel. It should be taken into account, that hydrolysis is carried out in a solution of slightly diluted organic acid, at pH 3,6÷6,5 and a temperature of about 68°C. As a raw material the legs of an immature poultry (birds) no older than 9 weeks are used (US-A-4455302).

The raw material used and also absence of the operations before hydrolysis, earmarked for its homogenization and purification from non-destroyed tissue elements with extraction of the supernatant, contained components of morphoplasm and glycocalyx of cells, do not afford to obtain desired product with high (more than 30%) concentration of mentioned components, and also to reduce immunodepressants, neutral and ballast component in its content.

There is another known preparation for the normalization of physiological state of human and animals, which includes a biologically active substance on the basis of organic compounds of the hydrolysis products from disintegrated animal tissues, that is used in healing traumatized parts of the body (US-A-4455302).

A disadvantage of this preparation is the fact, that it can be only used as local applications to injured parts of the body, consequently its efficacy is comparatively low.

The noted disadvantages are partially eliminated in analogous another known preparation with immunomodulating activity, designed for the normalization of physiological state of animals (SU-A-904707), which is administered intramuscularly at a dose of 0.75 to 1.0 ml/kg of the body weight with 3 day intervals between injections.

A disadvantage of the well known method is the fact, that medical preparation is to be administered into organism only as injections, and also that its comparatively low efficiency is because of the presence of immunodepressants in its composition. Besides, it can induce side effects (allergy, delayed hypersensitivity, etc.) because of the presence of immunogenes in its composition.

Significant disadvantage of this preparation is the presence of a great number of various biologically active substances in its composition, which causes, a danger of overdosages on the one hand and the development of side effects, that makes a desired preparation more expensive on the other hand.

There is a known method of normalization of physiological state of human and animals suffering from diseases caused by functional insufficiency of some organs and tissues. It suggests the administration of a preparation based on organic compounds of cell membrane components of animal tissue (SU-A-904707).

This known method is of low efficacy and requires to use high doses of a BAS (up to 10 mg/kg of the body weight). This creates a danger of overdosages and development of side effects.

DESCRIPTION OF THE INVENTION

The noted disadvantages are eliminated in the declared biologically active substance with immunomodulating activity, in the declared method of this BAS production, and in the preparation made on its basis, as well as in the method of its 0 use for the normalization of physiological state of the organism.

Making a biologically active substance having immunomodulating properties, a method of its production and a pharmacological preparation based on it ensuring a high biological activity without toxicity and various side effects, and also the possibility of obtaining desired product with determined content and determined properties is main task taken in the basis of invention.

The task set up is solved by the fact that a biologically active substance having immunomodulating properties, on the basis of organic compounds of the water-soluble hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue.

It should be taken into account, that the term of "morphoplasm" is used here in a wide significance and covers the structurized part of cellular cytoplasm, represented by cellular organelles, membranes, cytoskeleton and similar cellular structures (Jasuo Kagava. Biomembranes.-M. - "High School".-1985. -p.p.13,15), while the term of "glycocalyx" is used to designate a carbohydrate-containing layer adjoined to the exterior part of plasmatic membrane of a cell. -"Mir". -1986. -p.97).

The task is also solved by the fact that the declared BAS thermostable low-molecular mass components are present in an amount ranging from 30 to 100 percent by mass.

In addition, the declared BAS comprises, low-molecular mass components of morphoplasm and glycocalyx of cells, with a molecular mass ranging from 0.8 to 10.0 kDa.

The task set up is also solved by the fact that the declared BAS comprises organic compounds at the following ratio in per cent by mass:

| | |
|---|---|
| Polypeptides | up to 26.0; |
| Peptides | from 10.0 to 22.5; |
| Amino acids | from 32.0 to 60.1; |
| Carbohydrates | from 10.5 to 28.7; |
| Lipids, nucleotides and other organic compounds | from 2.0 to 11.0. |

In addition, the declared BAS comprises carbohydrates, in a composition of said organic compounds, at the following ratio in per cent by mass within:

| | |
|---|---|
| High-molecular mass compounds having a molecular mass of above 10.0 kDa | up to 2.0; |
| Low-molecular mass compounds having a molecular mass ranging from 0.8 to 10.0 kDa | from 2.5 to 11.5; |

(continued)

| Free monomers having a molecular mass of less than 0.8 kDa | from 6.0 to 17.0. |
|---|---|

In the declared BAS a ratio between the mass content of carbohydrates and that of peptides, within said organic compounds having a molecular mass ranging from 0.8 to 10.0 kDa, ranges from 0.25 to 0.6.

The task set up is also solved by the fact that the declared BAS comprises organic compounds at the following ratio in per cent by mass:

| Polypeptides | from 16.3 to 24.7; |
|---|---|
| Peptides | from 11.9 to 17.1; |
| Amino acids | from 32.0 to 48.2; |
| Carbohydrates | from 14.6 to 20.8; |
| Lipids, nucleotides and other organic compounds | from 5.1 to 9.3. |

In addition the declared BAS comprises organic compounds at the following ratio in percent by mass:

| Polypeptides | from 5.5 to 12.7; |
|---|---|
| Peptides | from 13.6 to 21.0; |
| Amino acids | from 37.1 to 52.9; |
| Carbohydrates | from 16.1 to 25.1; |
| Lipids, nucleotides and other organic compounds | from 5.3 to 25.1. |

The declared BAS comprises also organic compounds at the following ratio in percent by mass:

| Polypeptides | less than 0.3; |
|---|---|
| Peptides | from 14.9 to 22.5; |
| Amino acids | from 43.5 to 60.1; |
| Carbohydrates | from 15.5 to 28.7; |
| Lipids, nucleotides and other organic compounds | from 5.0 to 9.2. |

The task set up in this invention is also solved by the fact that a method for producing a biologically active substance having immunomodulating properties, on the basis of organic compounds of the hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue, comprising disintegrating the washed raw material of animal tissue with subsequent homogenization in physiological and/or buffer solutions, separating non-disintegrated tissue elements by means of centrifugation at 150 to 250 g for 1 to 15 min. and/or filtration, isolating supernatant, hydrolyzing said supernatant, separating the hydrolysis products by means of centrifugation at above 1 500 g for 20 to 40 minutes and/or filtration, and collecting of the water-soluble hydrolysis products as desired product.

The following raw material is selected from the group consisting of embryonic tissue to the exclusion of tissue from human embryos, differentiating thymocytes of the thymus, spermatogones and spermatocytes of the testes, placental tissue, epithelial tissue of the intestine, bone marrow cells, regenerating cutaneous tissue, regenerating hepatic tissue, tissue of regenerating amphibian limbs, pathologically transformed tissue taken prior to the stage of regeneration, and any combination thereof.

Besides, the disintegrating of raw material, prior to homogenization, is adjusted up to damaging integrity of the cells, with subsequent extraction of their content by means of an additional washing with filtration and/or centrifugation at 300 to 15 000 g for 10 to 20 minutes, followed by separating extracted disintegrated tissue.

The supernatant isolated following separating non-disintegrated tissue elements contains components of morphoplasm and glycocalyx of the cells in the composition of organic compounds from 30 to 60 percent by mass.

The hydrolysis is performed at a temperature ranging from 4°C to 45°C. This process can be done in the presence of a preservative.

The task set up is also solved by means of subjecting the hydrolysis products to thermal treatment at a temperature ranging from 60°C to 120°C up to complete denaturation of thermolabile compounds with subsequent filtration and/or centrifugation at above 1 500 g for 20 to 40 minutes with collecting supernatant containing thermostable components as desired product.

Besides, the hydrolysis products are subjected to fractionation by means of dialysis through a semipermeable membrane and/or ultrafiltration and/or gelfiltration with collecting a fraction of low-molecular mass components having a molecular mass less than 10.0 kDa as desired product.

The task set up in the invention is also solved by the fact that a preparation for the normalization of the physiological state of human and animal organisms, consisting of a biologically active substance having immunomodulating properties on the basis of organic compounds of the water-soluble hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue, said substance being taken in an amount ranging from 0.001 to 85.0 percent by mass with the rest being an excipient.

The task set up in the invention is also solved by the fact that the biologically active substance comprises organic compounds at the following ratio in per cent by mass:

| | |
|---|---|
| Polypeptides | up to 26.0; |
| Peptides | from 10.0 to 22.5; |
| Amino acids | from 32.0 to 60.1; |
| Carbohydrates | from 10.5 to 28.7; |
| Lipids, nucleotides and other organic compounds | from 2.0 to 11.0. |

The task set up in the invention is also solved by the fact that the biologically active substance comprises organic compounds at the following ratio in per cent by mass:

| | |
|---|---|
| Polypeptides | from 16.3 to 24.7; |
| Peptides | from 11.9 to 17.1; |
| Amino acids | from 32.0 to 48.2; |
| Carbohydrates | from 14.6 to 20.8; |
| Lipids, nucleotides and other organic compounds | from 5.1 to 9.3. |

The task set up in the invention is also solved by the fact that the biologically active substance comprises organic compounds at the following ratio in per cent by mass:

| | |
|---|---|
| Polypeptides | from 5.5 to 12.7; |
| Peptides | from 13.6 to 21.0; |
| Amino acids | from 37.1 to 52.9; |
| Carbohydrates | from 16.1 to 25.1; |
| Lipids, nucleotides and other organic compounds | from 5.3 to 11.0. |

The task set up in the invention is also solved by the fact that the biologically active substance comprises organic compounds at the following ratio in per cent by mass:

| | |
|---|---|
| Polypeptides | less than 0.3; |
| Peptides | from 14.9 to 22.5; |
| Amino acids | from 43.5 to 60.1; |
| Carbohydrates | from 15.5 to 28.7; |
| Lipids, nucleotides and other organic compounds | from 5.0 to 9.2. |

As an excipient the physiological solution of sodium chloride and/or saline buffer solution and/or carbohydrate compounds and/or fat of minerals, animals, vegetables or synthetic origin or any mixture thereof are present.

The preparation can also comprise a preservative.

The task set up in the invention is solved by the use of a biologically active substance having immunomodulating properties on the basis of organic compounds of the water-soluble hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue, for the manufacture of a preparation for the normalization of the physiological state of human and animal organisms, said preparation being suitable for administration to said organisms and consisting of said biologically active substance in an amount ranging from 0.001 to 85.0 percent by mass, with the rest being an excipient. The task set up in the invention is solved by the use of a preparation for administration as intramuscular injections at a dose ranging from 0.005 to 0.5 mg/kg of the body weight with 1 to 7 day intervals between injections and/or inhalations at a dose ranging from 0.012 to 0.12 mg/kg of the body weight with 4 to 48 hour intervals between inhalations and/or sublingual administrations at a dose ranging from 0.012 to 0.12 mg/kg of the body weight with 4 to 48 hour intervals between administrations and/or wet feedings at a dose ranging from 0.02 to 1.0 mg/kg of the body weight with 1 to 10 day intervals between feedings and/or oral pills at a dose ranging from 0.02 to 10 mg/kg of the body weight with 1 to 10 day intervals between administrations and/or intranasal administrations at a daily dose ranging from 0 to 0.05 mg/kg of the body weight with 2 to 24 hour intervals between administrations till the normalization of physiological parameters.

The task set up in the invention is solved by the use of a preparation for administration as applications to mucous or wound surfaces in the form of compresses, suppositories, ointments, powders and gels with 1 to 10 day intervals between applications till the normalization of physiological parameters.

At the same time, the normalization of physiological state of human and animals can be realized by means of stimulation of the immune system, mainly by activation of the reticuloendothelial system, T- and B-systems, neutrophiles and/or natural killers.

It has been established, that practical efficiency of the declared BAS, produced accordingly to the declared method of production lays in its immunomodulating activity.

To a great extent, it determines the strategy of declared BAS influence on the organism in case of the normalization of homeostasis.

By the directed activation of the immune system, and first of all the cells of macrophage-granulocyte row, the declared BAS and preparation on its basis ensure:

1) influence on non-concrete pathology, but at the same time on an improvement of whole status of physiological state of the organism, by means of the mobilization of internal reserves directed to the normalization of all homeostasis parameters and, among them to concrete pathology. At the same time concomitant declinations caused by pathology without any side effects are corrected. Normalizing parameters, the declared BAS, in comparison with many other pharmacological preparations, does not worse the homeostasis of an organism;

2) complex influence, which affords to widen spheres of BAS administration in some diseases, controlled by the immune system of an organism;

3) such degree of the effectiveness, directed to normalization of homeostasis parameters, which correlates with degree of deviation from the norm. In case of the absence of such deviations or the normalization of parameters during treatment, cell activity of the immune system, stimulated by a preparation, is normalized in some days after injection of the declared BAS without any consequences for the organism, and thus the effect of a BAS is stopped. At the same time the principle of feedback is realized: the less deviation the less effect is and vice versa.

The results mentioned are achieved owing to special properties of the declared BAS and preparation based on it which are absent in analogues. These properties are ensured by the fact that the hydrolysis products are present in a BAS content which contains of components of morphoplasm and glycocalyx of cells, modified during the processes of embryogenesis and/or prolyferation, and/or differentiation of cells, and/or pathology, preceding and/or accompanying regeneration and/or reparation of the tissue.

It is established, that the increased consistence of modified components of morphoplasm and glycocalyx of cells is ensured, by usage of animal tissues, taken on the stage of above mentioned processes such as embryogenesis and/or proliferation, and/or differentiation of the cells, and/or pathology, preceding and/or accompanying the process of regeneration and/or reparation of the tissue.

There are the following tissues of such a type:

- differentiating thymocytes of the thymus (the process of differentiation of cells);
- spermatogones and spermatocytes of the testes, cells of bone marrow, epithelial tissue of the intestine (the processes of proliferation and differentiation of the cells);
- embryonal tissue to the exclusion of tissue from human embryos, placental tissue (the processes of proliferation, differentiation, embryogenesis);
- tissues of regenerating limbs of amphibians, rat regenerating liver after partial hepatectomy or after a damage by

CCl$_4$, regenerating cutaneous tissue, pathologically altered tissue, for instance, in the case of gangrene, in cases of chemical or thermal injures (any combinations of the above mentioned processes).

The use of listed animal tissues as raw material in the declared method of BAS preparation, ensures BAS production with expected properties.

It was also established that a characteristic feature of above listed tissues is altered immunogenicity of their cell membranes that was defined by modifications of components of morphoplasm and glycocalyx of cells. This feature allows their differentiation from intact or stable cells of tissues in mature animals. This property of modified components of morphoplasm and glycocalyx of cells affords their usage in the declared BAS which, being administered to humans or animals, activate their immune system toward searching and eliminating pathology.

Physical carriers of information in the above listed processes and/or deviations from the norm in pointed tissues are specific components included into the structure of morphoplasm and glycocalyx of cells, and the structure is modified in case of changing physiological status of the cells. The declared BAS comprises modified components of morphoplasm and glycocalyx of cells, which activate directionally the immune system.

The process of washing a disintegrated tissue from the components of blood and tissue extract in combination with homogenization, with subsequent purification of non-destroyed tissue elements before the hydrolysis, ensures the high concentration of components of morphoplasm and glycocalyx of cells (30÷60%) in the declared BAS and preparation on its basis, and also a significant decrease in the consistence of immunodepressants, neutral (in respect of the specific activity) and ballast compounds, comprising the rest of mass in a desired product. It increases the directed immune reaction of an organism.

A response of the organism is augmented considerably following an injection of the declared BAS which comprises modified components of morphoplasm and glycocalyx of cells in the form of thermostable components stable at a temperature T≤120°C; in the form of low-molecular components, namely, with a molecular mass of M=800÷10,000 Da.

The declared BAS is used as an active ingredient in a preparation, where various excipients depending upon their prescription are presented.

At the same time, an unobvious dependence between effectiveness of the preparation and its BAS content is revealed. It allowed to reduce considerably, a content of active ingredient while keeping the necessary efficiency of preparation, and preventing a possibility of overdosages, side effects.

Besides, the above discribed properties of preparation afford its use for the normalization of physiological state of the organism at any doses, namely from 0.001 to some grams per 1 kg of the body weight.

The pointed properties are below illustrated in examples of concrete realization of the invention, particularly in Examples 1, 2, 4, 8, 9, 12, 16, 18-25 and others.

In the above described method of using a new preparation for the treatment of diseases mainly induced by functional insufficiency of organs and tissues is achieved owing to concrete schemes and ways of its administration. This is below illustrated in examples of concrete realization of the inventions, particularly in Examples 3, 4, 7, 8, 11-13, 15-17 and others.

For internal usage, the preparation is administered in the form of injections with a physiological solution of sodium chloride or buffer salt solution as an excipient. For external usage, besides above mentioned solutions as a excipient can be used components of gels, i.e.- carbohydrate compounds of animal as well as plant origin, for example honey, juices, pulp of plants, etc. Fats of minerals, animals, vegetables or synthetic origin or their mixtures can be used as excipient for ointments.

DETAILED DESCRIPTION OF THE INVENTION

According to the invention, a biologically active substance with immunomodulating properties, for the normalization of physiological state of the organism, is produced by the following method.

The animal tissue to the exclusion of tissue from human embryos modified in the processes of embryogenesis, and/or proliferation and/or differentiation of the cells, and/or pathology preceding and/or accompanying the process of regeneration and/or reparation of the tissue was taken as a raw material.

The pointed animal tissue to the exclusion of tissue from human embryos is washed and disintegrated with subsequent homogenization in the physiological and/or buffer solution up to complete destruction of the cells. At that time the homogenized mixture is purified from the non-destroyed elements of the tissue by filtration and/or centrifugation with isolating supernatant, contained components of morphoplasm and glycocalyx of cells, with following hydrolysis. After centrifugation and/or filtration, the hydrolysis products are used as a desired product for the normalization of physiological state of the organism, for external administration (inhalations, applications, intranasal drops, in ointment content and so on).

An efficiency of the desired product depends upon modified components of morphoplasm and glycocalyx of cells in its content. Their content can be elevated by choosing raw material with an increased intensiveness of the modification processes and optimal conditions of realization of the production method.

The choice of raw material depends upon the fact, that components of morphoplasm and glycocalyx of cells have very specific reaction to the changes of their structural organization, which results into changes of immunogenicity of tissue, and by this means, cause corresponding reactions of the immune system of an organism.

It is established, that immmunogenicity of modified structure is ensured on the stage of above named processes (embryogenesis and so on).

The following animal tissue can be used as raw material:

- differentiating thymocytes of the thymus;
- spermatogones and spermatocytes of testes, cells of bone marrow, the epithelial tissue of the intestine (the processes of proliferation and differentiation of cells);
- embryonal tissue to the exclusion of tissue from human embryos, placental tissue (processes of proliferation, differentiation, embryogenesis);
- tissues of regenerating limbs of amphibians, regenerating liver of rats after partial hepatectomy or after a damage induced by $CCl_4$, regenerating cutaneous tissue, pathologically altered tissue, for instance in the case of gangrene, in the cases of chemical or thermal injures (any combinations of the above mentioned processes).

The use of described animal tissues as raw material in the declared method of BAS preparation ensures obtaining a biologically active substance with the expected properties.

It was established that a characteristic feature of the above named varieties of tissues is their changed structure of components of morphoplasm and glycocalyx of cells. This affords their differentiation from intact or stable cellular tissues of mature specimens. This feature of indicated tissues allows their use in preparing the declared BAS which, being administered to humans or animals, activates their immune system toward searching and eliminating pathology.

It is preferably to use the animal tissue taken at the incremental stage of the process of modification. This stage can be difined empirically by a trend in changing immunogenicity. For example, in the case of embryonic tissue use, this stage constitutes the first half of embryogenesis. Regenerating liver of rats is used 4-12 hours after partial hepatectomy. In the case of use of regenerating limbs of amphibians, this corresponds to the blastema formation.

The increase of respective content (concentration) of components of morphoplasm and glycocalyx of cells may be achieved owing to the realization of such a regimen of the declared method, with removing non-destroyed elements of tissue, represented mainly by connective tissue, as an indispensable condition.

The purification of non-destroyed elements could be ensured by centrifugation in the range of accelerations of 150÷250 g for 10÷15 minutes, that can be explained by the fact, that at higher accelerations and more prolongated duration of the process there is precipitation of elements of morphoplasm and glycocalyx of cells, but at accelerations less than 150 g the purification is not effective.

The purification of non-destroyed elements could be ensured by filtration using big pore filter, for instance, through several layers of gauze, cotton (coarse calico) or through caprone nets.

In order to strengthen the immunomodulating properties of desired product, before homogenization the tissue is disintegrated up to damaging cell integrity with following extraction of their content by means of additional washing with physiological solution, and/or centrifugation at the acceleration 300÷15 000 g for 10÷20 minutes and, followed by separation of washing disintegrated tissue.

At that time, removing of significant part of immunodepressants, which are present in the content of blood serum and tissue extract is achived on the one hand and an increase in respective content of components of morphoplasm and glycocalyx of cells, that is very important for tissues with their low content - on the other hand.

The additional washing of used disintegrated animal tissue before the homogenization by centrifugation at the acceleration 300÷15 000 g for 10÷20 minutes affords the most qualitative separation of extracted tissue from extract under industrial conditions. The analogical effect may be achieved by filtration using big pore filter, for instance, through several layers of gauze, cotton (coarse calico) or through caprone nets.

The respective content of components of morphoplasm and glycocalyx of cells in tissue ingredients, taken for hydrolysis process is determined by protein and carbohydrate contents in cellular structures, free from non-destroyed tissue elements.

The efficiency of desired product rises even in a case of using components of morphoplasm and glycocalyx of cells in the form of low-molecular components, so far as it becomes devoid of side effects and requires a wider spectrum of action without being species-specific.

In order to do it, hydrolysis, decomposing high-molecular structural formations of morphoplasm and glycocalyx of cells into products comprising components with lower molecular mass was performed. The efficiency of the hydrolysis products will depend upon specificity of hydrolysis, which in its turn depends largerly upon temperature - time characteristics.

It was established, that immunomodulating properties of the declared substance, particularly its capability to increase activity of macrophages in rat peritoneal exudate in vitro, are maximum in a case of hydrolysis at a temperature ranging from 4°C to 45°C.

Any deviations from this range are undesired, because:

- at a temperature of less than 4°C a duration of hydrolysis increases that reduces the economic efficiency;
- at a temperature of above 45°C an activity of enzymes, ensuring hydrolysis of raw material decreases while decomposition processes of active components accelerate and, as a consequence, the quality of desired product is brought down.

A duration of hydrolysis is defined by the maximum level of immunomodulating effect of the declared BAS, by activation of macrophages of peritoneal exudate according to the NBT test in particular.

A prolongated hydrolysis has to be performed in the presence of a preservative.

An increase in relative content (relative concentration) of low-molecular components in the hydrolysis products comparing that of thermolabile compounds, is ensured by thermal treatment of hydrolyzate up to complete denaturation of thermolabile compounds. After it they should be removed in the form of sediment by the method of filtration and/or centrifugation at no less than 1,500 x g for 20 to 40 minutes. Supernatant comprising thermostable components is used as desired product.

The thermal treatment of the hydrolysis products is performed at a temperature ranging from 60°C to 120°C. Outside this range, BAS specific activity declines.

Thus, at a temperature less than 60°C, not all proteins can be denaturated, resulting in an increase of the content of thermolabile compounds of desired product. As a consequence, BAS specific activity declines.

At a temperature above 120°C, a jump like increase in the rate of structural changes in peptide and carbohydrate components of desired product takes place, that also declines its quality.

After thermal treatment of the hydrolysis product, thermostable components are separated by the method of centrifugation at speed no less than 1,500 x g for 20 to 40 min. so far as a degree of removing the denaturated thermolabile compounds at lower accelerations falls down considerably.

The coagulated denaturated thermolabile compounds are eliminated from supernatant by the method of filtration through a paper filter or cloth filter.

The desired product comprising thermostable components of cell membranes has a higher specific activity in comparison with supernatant of the hydrolysis products and allows solution of a wider spectrum of tasks. It can be used as injections in veterinary and medicine.

The following increase in specific activity of a BAS is achieved by means of collecting low-molecular components with a molecular mass less than 10,000 daltons from the hydrolysis productsby dialysis through a semipermeable membrane and/or ultrafiltration, and/or gelfiltration. This allows high-molecular compounds capable of inducing side effects to remove completely.

Thus a desired product is devoid of side effects and possesses high efficiency, which allows its wide use in clinical practice.

The declared BAS is used as an ingredient in producing a pharmacological preparation, where its concentration depends upon a way of its administration. When wet or soft feedings are used BAS concentration in the preparation is low and increases when a preparation is used as ointments, gels or powders.

Depending upon a dosage form of the preparation, various excipients are used. Thus, a physiological solution of sodium chloride and saline buffer solution are used in injections, applications, intranasal drops. Carbohydrate compounds are used as an excipient dosage form designed for feeding of bees (for instance, 10 % sugar syrup) and gels (for example, honey, juices, and pulp of plants). For ointments, fats of minerals, animals, vegetables or synthetic origin and their mixtures are used as an excipient.

Examples of concrete realization of the declared group of inventions, as well as the methods used for determination of BAS compound and properties, are described below.

Example 1

For the production of a declared biologically active substance, as raw material cattle embryonic tissue taken at the first half of pregnancy was used. After disinfection in a weak solution of potassium permanganate ($KMn_7O_4$), cutaneous and muscular tissues, liver, spleen, pancreas, kidneys, lungs, heart were cut off from embryons with their following disintegration.

Then 10 kg of disintegrated mass mixed with 30 kg of physiological solution with subsequent homogenization using homogenizer with knife-like blenders until homogeneous mass was obtained. Non-destroyed elements of tissue were removed from mixture by decantation, and received layer of liquid over sediment containing morphoplasm and glycocalyx of cells, was autolized at a temperature of T=45°C for 48 hours.

Obtained hydrolyzate was filtrated through paper filter, and filtrate, containing morphoplasm and glycocalyx of cells, was used as desired product. Content of organic compounds and specific activity induced by this product, are shown in Tables 1 and 2.

The desired product including a BAS in an amount of 1.0 per cent by mass, with the rest being an excipient, was mixed with 9 volumes of cucumber pulp and used as a pharmacological preparation in the form of facial gel mask for the cosmetic purposes. In the cases of long-term storage, a preservative has to be added into gel (nipagine and nipasole at a ratio of 5:1 respectively) up to a final concentration of 0.1 per cent by mass. Facial gel applications were made for 2÷3 hours. The procedure was repeated 1÷2 times per week. The beneficial effect controlled by skin state was noted following 4÷10 procedures.

Example 2

The embryonic tissue, obtained as in Example 1, was disintegrated. Then 10 kg of disintegrated mass mixed with 30 kg of physiological solution and homogenized until homogeneous mass was received. To remove non-destroyed elements of connective tissue, homogenized mass was subjected to centrifugation at acceleration 250 x g for 10 min. After centrifugation sediment was removed and produced supernatant was filtrated through cotton (coarse calico) in order to remove fluctuated fat. The preservative (chinozol) was added to homogenate, purified by this method, and contained 30% components of morphoplasm and glycocalyx of cells, up to the final concentration 0,08 per cent by mass. After mixing, homogenate was hydrolyzated at a temperature of T=4°C for 6 weeks.

The produced hydrolyzate was been centrifugated at 15 000 x g for 20 minutes with extraction of supernatant, containing the components of morphoplasm and glycocalyx of cells as desired product. The content of organic compounds in desired product and specific activity induced by this product are shown in Tables 1 and 2.

Table 1

| Immunomodulating and regenerating effects of biologically active substances (BASes) | | Quantitative indices of BAS effects in examples | | | |
|---|---|---|---|---|---|
| | | N 1 | N 2 | N 3 | N 4 |
| 1. An increase in the activity of macrophages of rat peritoneal exudate versus control,% | Enzyme activity (NBT-test) | 52,3 | 54,2 | 71,0 | 58,3 |
| | Phagocyte activity by E.Coli | 37,3 | 39,1 | 54,5 | 43,8 |
| 2. An increase in the activity of blood neutro phyles versus control, % | Enzyme activity (NBT-test) | 66,7 | 71,2 | 85,0 | 75,4 |
| | Phagocyte activity by E.Coli | 44,5 | 49,8 | 59,8 | 52,2 |
| 3. Stimulation of blasttransformation reaction versus control ,% | T-lympho cytes | 40,2 | 44,8 | 51,3 | 46,2 |
| | B-lympho cytes | 48,7 | 50,2 | 61,0 | 55,2 |
| 4. An increase in titre of thymic serum-like factor (TSF) versus control % | | 156,3 | 167,4 | 249,8 | 210,4 |
| 5. Stimulation of natural killer activity of rat splenocytes versus control,% | | 65,7 | 66,1 | 78,5 | 72,3 |
| 6. An increase in reparative abilities of rat hepatocytes by reconstruction of activity after $CCl_4$ injection versus control,% | Na,K-ATP-ases | 20,1 | 24,3 | 29,1 | 26,7 |
| | Blood aminotranspherases | 18,7 | 22,1 | 32,3 | 27,4 |
| | $K^+$ Concentration of hepatocytes | 10,3 | 13,8 | 19,8 | 16,8 |

12

Continuation of Table 1

| Immunomodulating and regenerating effects of biologically active substances (BASes) | | Quantitative indices of BAS effects in examples | | | | | |
|---|---|---|---|---|---|---|---|
| | | N 7 | N 8 | N 9 | N 11 | N 12 | N 13 |
| 1.An increase in the activity of macrophages of rat peritoneal exudate versus control,% | Enzyme activity (NBT-test) | 102,6 | 73,0 | 87,6 | 158,2 | 127,0 | 151,1 |
| | Phagocyte activity by E.Coli | 73,9 | 54,7 | 64,1 | 124,7 | 116,1 | 122,4 |
| 2.An increase in the activity of blood neutrophyles versus control, % | Enzyme activity (NBT-test) | 109,2 | 81,6 | 95,4 | 179,5 | 138,3 | 167,6 |
| | Phagocyte activity by E.Coli | 78,9 | 61,9 | 60,2 | 134,3 | 112,9 | 125,4 |
| 3. Stimulation of blasttransformation reaction versus control, % | T-lymphocytes | 79,7 | 59,3 | 69,2 | 149,3 | 120,3 | 141,3 |
| | B-lymphocytes | 93,3 | 71,9 | 83,4 | 189,6 | 146,8 | 176,2 |
| 4.An increase in titre of thymic serum-like factor(TSF) versus conrol, % | | 364,3 | 273,1 | 307,4 | 706,0 | 543,4 | 650,8 |
| 5.Stimulation of natural killer activity of rat splenocytes versus control,% | | 120,6 | 90,0 | 105,0 | 211,0 | 178,4 | 194,4 |
| 6.An increase in reparative abilities of rat hepatocytes by reconstruction of activity after $CCl_4$ injection versus control,% | Na,K-ATPases | 34,6 | 28,2 | 30,8 | 64,8 | 50,4 | 60,2 |
| | Blood aminotranspherases | 53,6 | 38,8 | 45,2 | 82,0 | 59,2 | 75,6 |
| | $K^+$ Concentration of hepatocytes | 27,0 | 22,2 | 24,6 | 52,5 | 43,9 | 50,4 |

Table 2

| Organic compounds | Content of organic compounds in BAS produced in examples mass. % | | | |
|---|---|---|---|---|
| | N 1 | N 2 | N 3 | N 4 |
| Polypeptides | 26,0 | 16,3 | 21,8 | 20,5 |
| Peptides | 10,0 | 11,6 | 20,0 | 11,9 |
| Amino acids | 51,5 | 48,2 | 32,0 | 44,3 |
| Content of carbohydrates<br>-high-molecular compounds with a molecular mass $M>10,0$ kDa | 2,0 | 0,3 | 1,1 | 0,6 |
| -low-molecular compounds with a molecular mass $M=0,8\div10,0$ kDa | 2,5 | 3,3 | 10,3 | 5,0 |
| -free monomers or compounds with a molecular mass $M<0,8$ kDa | 6,0 | 11,0 | 9,4 | 12,6 |
| I N   A L L : | 10,5 | 14,6 | 20,8 | 18,2 |
| Lipides, nucleotides and other organic compounds | 2,0 | 9,3 | 5,4 | 5,1 |
| T O T A L : | 100,0 | 100,0 | 100,0 | 100,0 |
| Correlation between carbohydrate and peptide contents G | 0,25 | 0,28 | 0,52 | 0,42 |

Continuation of Table 2

| Organic compounds | Content of organic compounds in BAS produced in examples mass.% | | | | | |
|---|---|---|---|---|---|---|
| | N 7 | N 8 | N 9 | N 11 | N 12 | N 13 |
| Polypeptides | 11,0 | 5,5 | 12,7 | less than 0,2 | 0,3 | 0,3 |
| Peptides | 21,0 | 13,6 | 18,6 | 19,1 | 14,9 | 22,5 |
| Amino acids | 37,1 | 52,9 | 47,3 | 43,5 | 60,1 | 49,8 |
| Content of carbohydrates –high-molecular compounds with a molecular mass M>10,0 kDa | 0,6 | 0,2 | 0,4 | less than 0,2 | 0,2 | 0,2 |
| –low-molecular compounds with a molecular mass M=0,8÷10,0 kDa | 11,0 | 4,0 | 7,4 | 11,5 | 4,9 | 11,0 |
| –free monomers or compounds with a molecular mass M<0,8 kDa | 13,5 | 12,8 | 8,3 | 17,0 | 10,4 | 11,2 |
| IN ALL: | 25,1 | 17,0 | 16,1 | 28,7 | 15,5 | 22,4 |
| Lipides, nucleotides and other organic compounds | 5,8 | 11,0 | 5,3 | 8,5 | 9,2 | 5,0 |
| TOTAL: | 100,0 | 100,0 | 100,0 | 100,0 | 100,0 | 100,0 |
| Correlation between carbohydrate and peptide contents G | 0,52 | 0,30 | 0,40 | 0,60 | 0,33 | 0,50 |

G – the coefficients, characterising the relative consistence of carbohydrates in the compounds with a molecular mass M=0,8÷10,0 kDa in respect to peptides

The desired product, consisting of a BAS in the quantity of 1,0 per cent by mass, with the rest being an excipient, was used as pharmacological preparation for increasing a survival of the weak younger animals of the fur-bearing animals, and also chickens of poultry in the form of inhalations in the special chamber, where animals were maintained for 15÷20 minutes. The preparation was sprayed at the rate of 0,5 ml for 1 $m^3$ during 1 minute. The process was repeated every 7 days. The effect controlled by a decrease in mortality level of younger animals was reduced by 30÷40%.

Example 3

Embryonal tissue, obtained as in Example 1, was disintegrated. Then disintegrated mass was flooded by two volumes of physiological solution and mixed. The mixture was been centrifugated at 1 500 x g for 20 min. The sediment was separated, mixed again in two volumes of physiological solution and centrifugated under the same conditions. The operation of washing of disintegrated mass was processed 3 times. Then 10 kg of washed disintegrated mass was mixed with 30 kg of centimolar phosphate salt buffer solution (0,01 M PBS) pH 7,2, which consists of centimolar sodium hydrogen phosphate and fifteen molar solution of sodium chloride (0,01 M $Na_2HPO_4$ И 0,15 NaCl), and was homogenized using coloidal mill until homogeneous mass was received. The non-destroyed tissue elements in the form of sediment were removed after centrifugation at 150 x g for 15 minutes. After removing fluctuated fat by filtration through cotton fibre (coarse calico), supernatant was treated by preservative with final concentration of 0,08 per cent by mass. After mixing, homogenate, consisted of 60 per cent by mass of components of morphoplasm and glycocalyx of cells was hydrolyzed at a temperature of T=4°C for 6 weeks.

The produced hydrolyzate was centrifugated at 1 500 x g for 40 minutes with subsequent extraction of supernatant, containing components of morphoplasm and glycocalyx of cells as desired product. Content of organic compounds and specific activity induced by this product are displayed in Tables 1 and 2.

The desired product, containing a BAS in the quantity of 1,0 per cent by mass, and with the rest being an excipient, has been used as pharmacological preparation in the treatment of calf parainfluenza in the form of intramuscular injections at a dose of 0,05 ml/kg of the body weight every third day. The pharmacological curative effect controlled by the body temperature and the presence of virus in blood was noted in 2÷6 injections.

### Example 4

The rough disintegrated mass (minced by mincing - machine) obtained as in Example 1, was flooded by two volumes of 3% acetic acid and mixed. The mixture has been filtrated through the cotton fibre under the vacuum conditions on nutch-filter. The sediment was taken out, mixed again in two volumes of physiological solution and centrifugated at 300 x g for 20 minutes.

Then 10 kg of the washed mass was mixed with 30 kg of centimolar phosphate salt buffer solution (0,01 M PBS) pH 7,2, which consists of centimolar sodium hydrogen phosphate and fifteenmolar solution of sodium chloride (0,01 M $Na_2HPO_4$ and 0,15 M NaCl), and was homogenized using coloidal mill until homogeneous mass was obtained. In order to romove non-destroyed elements of the tissue, homogenized mass was filtrated through the cotton fibre (coarse calico). The preservative in final concentration 0,08 per cent by mass was added into produced filtrate. After mixing, the homogenate, consisted of 50 per cent by mass of components of morphoplasm and glycocalyx of cells was hydrolyzed at a temperature of T=20°C for 8 days. The following operations were performed as in Example 3.

The consistence of organic compounds in desired product and the indices of its specific activity, determined in experiments, are shown in Tables 1 and 2.

The desired product, containing a BAS in the quantity of 1,0 per cent by mass, and with the rest being an excipient, was used as pharmacological preparation in the treatment of some dermatoses in the form of ointment, that was prepared by mixing lanoline, vaseline and BAS desired product in proportion of mass parts 1:3:1 respectively. The ointment was rubbed into the skin everyday. The curative effect controlled by state of skin has been shown in 4÷12 days.

### Example 5

The respective content of components of morphoplasm and glycocalyx of cells in the percentage by mass with respect to components of connective tissue and extract in tissue ingredients, separated for processing hydrolysis, were defined by the content of proteins and carbohydrates in cellular structures, free from non-destroyed elements of tissue.

In order to do it, homogenate, purified from non-destroyed elements of the tissue, as in Examples 1÷4, was ultra-centrifugated at 100 000 x g for 60 minutes.

The proportion of protein quantity in whole sediment and supernatant shows the proportion, of components of morphoplasm and glycocalyx of cells to extract proteins by protein.

Protein content in the sediment and supernatant was measured after ultracentrifugation of homogenate. After removing a supernatant, 4,9 ml of physiological solution were added to 0,1 ml of the sediment, and after resuspending just 0,1 ml was taken for determining protein quantity photometrically according to the SDS-Lowry method by intensity of solution colour (U.R.Laemmli.- Nature,-1970, -v.227, -5259, - p.680-685).

In order to determine protein quantity in the supernatant, 0,1 ml of supernatant was added into 0,9 ml of physiological solution and, after mixing, 0,1 ml were taken for determining protein quantity by SDS-Lowry method.

The content of components of connective tissue was defined by the amino acid analysis, according to the presence of oxylisine and oxyproline. In contrast to others well known BASes the declared one did not consist of these amino acids according to the control.

In order to determine a component content of glycocalyx of cells, the quantity of carbohydrates in homogenate, including compounds with a molecular mass above 800 dalton, according to the Antron reaction, after removing glycogen granules from homogenate was measured (S. Seifter, S.Dayton, C. Hovic. - Arch. Biochemistry and Biophysics, -1950, -25, -1, -p.191-200).

For this purpose 20 ml of tissue homogenate were layeredon on 5 ml of saccharose with d=1,40 density in ultracentrifuge test-tubes consisted of 30 ml, and ultracentrifugated at 100 000 x g for 60 minutes. Glycogen sediment of bottom in test-tubes, was removed and elements of morphoplasm and glycocalyx of cells were collected at interphase and after washing in physiological solution from saccharose by centrifugation were used for the analysis.

Determining a carbohydrate quantity of examined samples, general content of carbohydrates after hydrolysis, and that of free carbohydrates in the form of monomers was taken into account. The quantity of free carbohydrates in the content of the various compounds was determined by the difference of significances.

In order to carry out general hydrolysis, 1,0 ml of examined sample was taken and mixed with 1 ml of tetranormal hydrochloric acid (4,0 N HCl). The hydrolysis was carried out at a temperature of 105°C for 2 hours. After that the hydrolyzate was evaporated in the exsiccator with dry sodium hydroxide (NaOH) in vacuum. The dried probe was diluted in 1,0 ml of distilled water, and used, for analysis.

For determining the number of free carbohydrates in a BAS directly or after acid hydrolysis, the process was realized as follows: 1 ml of probe was mixed with 1 ml of distilled water. Then, 4 ml of 0,2 % Antron reagent, prepared in 95% sulphuric acid ($H_2SO_4$) were added with constant stirring at a temperature of -5°C. The produced mixture was boiled at a temperature of 95°C for 10 minutes and after cooling optical density was determined at the wave length of 620 nm. Glucose in the concentration of 20 mkg/ml was used as a standard.

During protein analysis of a BAS, obtained in Examples 1 and 2, the content of components of morphoplasm and glycocalyx of cells consisted of 37.6±7,4%, and of a BAS obtained in the Examples 3 and 4 - 51,8±8,2%.

Hereinafter in the text and in represented Tables, significances of indices determined in experiments, are shown as mean values calculated according to Student's test.

## Example 6

The hydrolyzate supernatant, obtained as in Example 1, was subjected to thermal treatment at various temperatures of heating $T_1$ =55°C, $T_2$=60°C, $T_3$ =95°C, $T_4$ =120°C and $T_5$ =135°C. The thermal treatment was performed up to complete denaturation of thermolabile compounds in composition of the hydrolysis product. Then, centrifugation was performed at above 1,500 x g for 30 min. with collecting the supernatant, comprising thermostable components of morphoplasm and glycocalyx of cell as desired product. In each case the desired product possessed immunomodulating effect whose value is shown in Table 3 as specific activity (activation of macrophages) in dependence upon the chosen range of heating temperatures of hydrolyzate. The optimal temperature of boiling, T=95°C, ensures a maximum increase in enzyme and phagocyte activity of desired product up to 72.8±4.8% and 64.3±5.6%, respectively. At a temperature of 55°C, an increase in specific activity of macrophages, was equal, on average to 57.3±5.3% (by NBT-test) and 43.0±3.4% (by phagocytosis). At a temperature above 120°C, an jump-like increase in rate of structural changes in peptides and carbohydrate-containing compounds, which resulted in structural changes in components of desired product was noted. This reduces BAS specific activity.

Thus, at a temperature of 135°C an increase in specific activity of a BAS is equal, on average, to 68.4±5.0% - by NBT -test and 66.2±6.1% - by phagocytosis. The maximum value of BAS specific activity is ensured at a temperature ranging from T=60-120°C. At that temperature, an increase in activity of macrophages for peritoneal exudate of rats (in vitro) is equal, on average, to 72.8±4.8% - by an increase in enzyme activity (NBT-test), and 64,3±5,6% - by an increase in phagocyte activity (according to E.Coli).

## Example 7

The hydrolyzate supernatant, obtained in Example 3, was subjected to thermal processing at a temperature of boiling $T_1$ =95°C. The boiling was processed up to complete denaturation of thermolabile compounds with their following centrifugation

Table 3

| Temperature of heating (thermoprocess) °C | Activation of macrophages of rat perito neal exudate (in vitro) as a result of influence of the declared BAS, obtained at various thermoprocesses versus control, % | |
|---|---|---|
| | Increase of enzyme activity NBT-test | Increase of phagocyte activity by E. Coli |
| 55 | 61,1 ± 2,2 | 52,3 ± 2,8 |

Table 3 (continued)

| Temperature of heating (thermoprocess) °C | Activation of macrophages of rat perito neal exudate (in vitro) as a result of influence of the declared BAS, obtained at various thermoprocesses versus control, % | |
|---|---|---|
| | Increase of enzyme activity NBT-test | Increase of phagocyte activity by E. Coli |
| 60 | $65,7 \pm 3,8$ | $55,1 \pm 2,1$ |
| 95 | $72,8 \pm 4,8$ | $64,3 \pm 5,6$ |
| 120 | $64,3 \pm 5,1$ | $58,3 \pm 4,2$ |
| 135 | $57,3 \pm 5,3$ | $43,0 \pm 3,4$ |

Differences are significant with control $P<0,05$

Table 4

| Number of fraction | Molecular mass of fractions M, KDa | Activation of macrophages of rat peritoneal exudate under influen ce of low-molecular fractions of declared BAS versus control, % |
|---|---|---|
| 1 | $M_1 >50,0$ | $52,1 \pm 4,8$ |
| 2 | $10,0<M_2<50,0$ | $93,8 \pm 7,6$ |
| 3 | $M_3 \leq 10,0$ | $148,4 \pm 17,4$ |
| 4 | $M_4 \leq 5,0$ | $160,7 \pm 15,1$ |
| 5 | $M_5 \leq 1,0$ | $120,5 \pm 11,7$ |
| 6 | $M_6 \leq 0,8$ | $87,3 + 8,7$ |
| 7 | $M_7 \leq 0,5$ | $53,4 \pm 7,2$ |

Differences are significant with control $P<0,05$

at 1 500 x g for 40 minutes after cooling. The sediment in the form of denaturated compounds was removed. The supernatant, comprising some thermostable components in the quantity of 60 per cent by mass according to the hydrolysis products of the water-soluble compounds by peptides was used as desired product before thermal processing.

The content of organic compounds in the hydrolysis products and quantitative indices of BAS effects are shown in Tables 1 and 2.

The desired product, containing a BAS in the quantity of 0,5 per cent by mass with all the rest being an excipient, was used as pharmacological preparation in the treatment of cow mastitis by intramuscular injections at a dose of 0,05 ml/kg of the body weight every third day. The curative effect controlled by the state of udder and the quantity of purulent discharges in the nipples was noted after 1÷4 injections.

Example 8

The hydrolyzate obtained in Example 2, was subjected to thermal processing at a temperature of boiling $T_1$ =95°C. The boiling was processed up to complete denaturation of thermolabile compounds with their following centrifugation at 50 000 x g for 20 minutes after cooling. The sediment in the form of denaturated compounds was removed. The supernatant, comprising some thermostable components in the quantity 30 per cent by mass according to the hydrolysis products of the water-soluble compounds by peptides was used as desired product before thermal processing.

The content of organic compounds in the hydrolysis products and quantitative indices of BAS effects are shown in Tables 1 and 2.

The produced desired product, containing a BAS in the quantity of 0,5 per cent by mass, and with the rest being an excipient, has been used as pharmacological curative preparation in prophylaxis of calf parainfluenza. The intramuscular injections at a dose of 0,05 ml/kg of the body weight were administered twice with 14 day intervasl between the injections. The effectiveness of preparations used has been registered by total quantity of animal diseased during the month. That quantity did not exceed 10% in experimental group, while it reached up to 22÷28% in control group.

Example 9

The hydrolyzate supernatant, obtained in Example 3, was subjected to thermal processing at a temperature of boiling $T_1 = 95°C$. The boiling was processed up to complete denaturation of thermolabile compounds with their following filtration through filtrating paper using a nutch - filter in vacuum after cooling. The filtrate, comprising some thermostable components in the quantity of 45 per cent by mass according to the hydrolysis products of the water-soluble compounds by peptides was used as desired product before thermal processing.

The content of organic compounds in the hydrolysis products and quantitative indices of BAS effects are shown in Tables 1 and 2.

The desired product, was dried by the method of lyophilization and, then, used in sublingual administration as curative preparation in female mastitis. The procedure was repeated everyday. The curative effect controlled by the breast state (the presence of infiltrations, suppurations) and also by blood analysis was noted in 1÷4 days.

Example 10

The hydrolyzate supernatant, obtained in Example 1, was fractionated using the ultracentrifuge "Amicon" with ultrafilters which allow passing the compounds with a molecular mass less or equal 50,000 Da; 10,000 Da; 5,000 Da; 1,000 Da; 800 Da and 500 Da.

The biological activity of obtained fractions, which consist of compounds with a molecular mass: 1) $M_1 > 50,000$ Da; 2) $10,000$ Da$< M_2 < 50,000$ Da. 3) $M_3 < 10,000$ Da; 4) $M_4 < 5,000$ Da; 5) $M_5 < 1,000$ Da; 6) $M_6 < 800$ Da and 7) $M_7 < 500$ Da, - was analysed by their ability to activate macrophages of rat peritoneal exudate according to NBT-test. The results of researches are shown in Table 4.

The analysis of Table 4 allowed to make the following conclusions:

1) distribution of specific activity according to fractions has its pronounced maximum, which lays in the range of a molecular mass from 800 up to 10,000 Da;
2) decrease of specific activity in the range of a molecular mass M>10,0 kDa is connected with an increase in inhibitor action of desired product and a decrease in the concentration of an active substance;
3) decrease of specific activity in the range of a molecular mass M<0,800 Da is connected with the reduction of active substance concentration represented as oligomer combinations with a molecular mass M>0,800 Da.

Example 11

The hydrolyzate supernatant, obtained in Example 3, was ultrafiltrated using hollow fibres ВПу-15-ПА (TY 6-12-151-87) of an apparatus AP-2,0. The produced ultrafiltrate, including low-molecular components of morphoplasm and glycocalyx of cell with a molecular mass less or equal M≤10,00 Da in the quantity of 60 per cent by mass according to the hydrolysis products of the water-soluble compounds by peptides was used as desired product before ultrafiltration.

The content of organic compounds in the hydrolysis products and quantitative indices of BAS effects are shown in Tables 1 and 2.

The desired product, containing a BAS in the quantity of 0,1 per cent by mass, and with the rest being an excipient, was used as pharmacological preparation in the treatment of rat experimental hepatitis with intramuscular injections at a dose of 0,05 ml/kg of the body weight every second day (the character and results of more detailed treatment are given in Examples 34 and 35).

Example 12

The hydrolyzate supernatant, obtained in Example 2, was subjected to dialysis for 16÷24 hours through cellophane membrane against a physiological solution with the following terms (0,8 g of the preservative per 1 kg of physiological solution of sodium chloride). The obtained dialysate was used as desired product, containing low-molecular components of morphoplasm and glycocalyx of cell with a molecular mass M≤10,00 Da in the quantity of 30 per cent by mass according to the hydrolysis products of the water-soluble compounds by peptides before the dialysis.

The content of organic compounds in the hydrolysis products and quantitative indices of BAS effects are shown in Tables 1 and 2.

The desired product, containing a BAS in the quantity of 0,1 per cent by mass, and with the rest being an excipient, was used as pharmacological preparation for the normalization of calf homeostatic parameters by intramuscular injections, administered at a dose of 0,05 ml/kg of the body weight every 1÷2 months with deviation from the norm. The curative pharmacological effect controlled by monthly animal weighting testifies about 45÷70% rising of

daily weight in comparison with untreated animal of control group.

Example 13

The hydrolyzate supernatant, obtained in Example 1, was subjected to gelultrafiltration in a Sephadex G-25 on the column with a diameter d=26 and length h=600 mm, which was equilibrated by decimolar phosphate buffer pH 7,2, included fifteencentimolar solution of sodium chloride (0,15 M NaCl). The fractionating of hydrolyzate supernatant according to molecular mass, was carried out by the method of column elution with the same buffer solution. The chromatographic profile of an eluate was controlled spectrophotometrically at the wave length of 280 nm as condition. The fraction of low-molecular components of hydrolyzate, comprising components of morphoplasm and glycocalyx of cell, was eluated as desired product.

The content of organic compounds in the hydrolysis products and quantitative indices of BAS effects are shown in Tables 1 and 2.

The desired product, containing a BAS in the quantity of 0,1 per cent by mass, and with the rest being an excipient, was used as curative pharmacological preparation in the treatment of calf bronchopneumonias or acute respiratory diseases (ARD), by intramuscular injections, which were administered every third day at a dose of 0,05 ml/kg of the body weight. The curative pharmacological effect controlled by body temperature has been shown in 1÷7 injections.

Example 14

In order to define how an efficiency of the declared BAS depends upon its content of some modified components of morphoplasm and glycocalyx of cell, the raw material from animal tissues was used. In these animal tissues the processes of embryogenesis and/or proliferation and/or differentiation of the cells, and/or pathology, preceding and/or accompanying the process of regenaration and/or reparation of the tissue taken place. The obtained tissues were processed as in Example 7.

The properties and effect of desired product, produced in these processes, with above mentioned modification of cell stuctures, namely morphoplasm and glycocalyx of cell are shown in Table 5.

The analysis of Table 5 shows, that the presence of immunomodulating effect and considerable increase of this effect in the declared substance depends upon used raw material, from which the hydrolysis products including some modified components of morphoplasm and glycocalyx of cell are obtained.

Thus, for this process the following raw material from animal tissue, taken precisely at the stage of cell proliferation and differentiation: spermatogones and spermatocytes of rat testes, cells of bone marrow, intenstinal epithelial tissue was used; and rat embryonic tissue was used as raw material with the processes of the embryogenesis. As raw material with pathologic processes, preceding regeneration, the tissue of rat regenerating liver after partial hepatectomy, was used. And with pathology, following regeneration - the tissue of regenerating axolotl (Ambistoma) limbs, the tissue of rat regenerating liver after subcutaneous injections of carbon tetrachloride ($CCl_4$) were used.

For comparison simultaneously with obtained experimental data of Table 5, the results of immunomodulating effect received during usage of muscular and liver tissue of intact mature male rats as raw material are illustrated.

The analysis of the data in Table 5 shows, that

Table 5

| Variety of used animal tissues | Activation of macrophages of rat peritoneal exudate (in vitro) as a result of influence of the declared BAS - TS, obtained at various tissues versus control,% | |
|---|---|---|
| | Increase in enzyme activity (NBT-test) | Increase in phagocyte activity by E. Coli |
| Hepatocytes of the intact liver | $32,2 \pm 5,4$ | $30,8 \pm 2,7$ |
| Intact muscular tissue of the mature animals | $43,6 \pm 6,8$ | $35,2 \pm 2,6$ |
| Spermatogones and spermatocytes of the testes | $63,8 \pm 3,5$ | $55,4 \pm 3,2$ |
| Epithelial tissue of the intestine | $68,2 \pm 6,3$ | $61,3 \pm 5,2$ |
| Liver hepatocytes after $CCl_4$ injection | $72,1 \pm 4,2$ | $64,4 \pm 4,3$ |

Table 5   (continued)

| Variety of used animal tissues | Activation of macrophages of rat peritoneal exudate (in vitro) as a result of influence of the declared BAS - TS, obtained at various tissues versus control,% | |
|---|---|---|
| | Increase in enzyme activity (NBT-test) | Increase in phagocyte activity by E. Coli |
| Cells of bone marrow | $74,2 \pm 6,1$ | $67,5 \pm 4,1$ |
| Tissue of regenerating limbs of axolotle* | 82,5 87,3 | 70,2 77,4 |
| Embryonal muscular tissue | $83,7 \pm 5,2$ | $73,6 \pm 4,8$ |
| Hepatocytes of rat regenerating liver after the hepatectomy | $94,8 \pm 5,7$ | $78,8 \pm 5,6$ |

Differences are significant with control P<0,05
* The tissue for which all significances displayed in this Table are obtained in concrete experiment

immunomodulating effect of the declared BAS using tissues, taken in the processes of embryogenesis, proliferation and pathology, preceding and/or accompanying the regeneration and/or reparation, is considerably higher, than in case of usage of other well known substances, because, in these well kown preparations a raw material used from animal tissue, in which the above mentioned processes were absent.

Besides, one can concluded, that the most strong immunomodulating effect was observed in regenerating tissues of the liver, axolotl (Ambistoma) limbs and embryonic tissue in comparison with the tissues of the above mentioned processes.

Example 15

For preparing the declared substance rat regenerating liver at the 4÷12th hour after hepatectomy, performed according to the well known method / G.M. Higgins, R.M. Anderson. - Experimental pathology of the liver. - Arch. Pathology, -1931, -N 12, -p. 186 / was used as raw material.

The disintegrated liver tissue was mixed with three volumes of physiologicacl solution, with subsequent centrifugation at 15 000 x g for 10 minutes, and the obtained sediment was mixed with three volumes of physiological solution and centrifugated under the same conditions. Washing of disintegrated liver tissue has been repeated 3 times and then the following operations consisted of obtaining desired product were processed as in Examples 3, 7 and 11.

The specific activity of obtained desired products was defined experimentally by the activation of reparative abilities in rat liver hepatocytes after their damage induced by carbon tetrachloride ($CCl_4$). As a result of that damage the activity of Na, K-ATPase was reduced by:

32,7±3,1% in case of usage of the desired product obtained as in Example 3;
43,6±3,9% - as in Example 7;
85,4±7,8% - as in Example 11;

in comparison with control group of untreated animals by the declared substance.

The activity of blood aminotranspherases was regenerated correspondingly by: 37,4±2,8% as in Example 3; 47,2±3,4% (7) and 98,3±1,7% (11), and potassium concentration of liver hepatocytes was regenerated by: 22,6±1,8% (3); 28,9±2,5% (7) and 59,1±4,7% (11), respectively.

The desired product, including a BAS in the quantity of 0,1 per cent by mass, and with the rest being an excipient, was used as curative pharmacological preparation in the treatment of calf hepatitis by intramuscular injections. The injections were administered at a dose of 0,05 ml/kg of the body weight every 3rd day with deviation from the norm. The curative pharmacological effect controlled by the indices of aminotranspherases, blood bilirubin and nitrogen concentration was shown after 7÷14 injections.

Example 16

For preparing declared substance, oilcake of bovine testes was used. This oilcake is obtained after lydase extraction with 3% acetic acid from tissue homogenate.

The obtained testicular oilcake, being a waste of lydase production, was repeatedly homogenized with triplicated volume of physiological solution using the knives-like homogenizer and centrifugated at 250 x g for 15 minutes in order to remove non-disintegrated elements of the tissue.

Using decinormal water solution of dry sodium hydroxide (0,1N NaOH), the acidity of medium was adjusted up to pH 7,4 and a preservative in the quantity of 0,8 gramm/Litr was added to homogenate, comprising components of morphoplasm and glycocalyx of cells, modified in the processes of proliferation and differentiation of cells, and after mixing it was hydrolyzed with subsequent processing as in Examples 3, 7 and 11.

The specific activity of obtained desired products was defined experimentally by the indices of macrophage activation in peritoneal exudate and neutrophiles of rat blood. Using a desired product, obtained as in Example 11, enzyme activity of macrophages was increased by 108,3±13,2%, phagocyte activity according to E.Coli - by 92,2±10,2%. For blood neutrophiles these indices were increased by 99,2±14,1% and 72,4±8,3% correspondingly.

The desired product, including a BAS in the quantity of 1,0 per cent by mass, and with the rest being an excipient, mixed with 50 volumes of water was used as pharmacological curative preparation in the form of drinks for the normalization of parameters from hen homeostasis. The hens were received drink as a wet feedings at a dose of 5 ml/kg of the body weight every 2÷6 weeks with deviation of the pointed parameters from the norm.

An efficiency was controlled by the calculation of eggs-laying, that testified of its rising by 15÷30% comparing with control group of hens untreated with this preparation.

Example 17

For preparing a declared substance, cow placental tissue, processed as in Examples 3, 7, 11, was used.

The specific activity of obtained desired products was defined experimentally by macrophage activation of peritoneal exudate and neutrophiles of rat blood, comparing with control group. During the usage of a desired product, obtained as in Example 11 an enzyme activity of macrophages was increased by 150.6±17,4%, and phagocyte activity - by 109,3±12,7% according to E.Coli. For blood neutrophiles these indices were increased by 137,6±16,8% and 95,7±11,3% correspondingly.

The desired product, including a BAS in the quantity of 0,5 per cent by mass, and with the rest being an excipient, mixed with 50 volumes of water was used as pharmacological preparation in the treatment of cow endometritis by administration of intramuscular injections. The preparation at a dose of 0,05 ml/kg of the body weight was injected every 3rd day. The curative pharmacological effect controlled by the parameters of inflammatory reaction was shown after 4÷8 injections.

Example 18

For preparing a declared substance, a oilcake of the thymus was used. This oilcake was obtained after the extraction of some insoluble tissue components from the homogenate that were wastes of thymosine production.

The thymic oilcake was processed as in Examples 3, 7 and 11.

The specific activity of obtained desired products was defined experimentally by the stimulation of blast-trasformation reaction in T-lymphocytes. In the case of usage of a desired product, obtained as in Example 11, it was increased by 165,6±13,7% comparing with control group.

The desired product, including a BAS in the quantity of 0,5 per cent by mass, and with the rest being an excipient was used as curative pharmacological preparation in infectious diseases for intranasal administration as prophylactic means.

The preparation was administered in the quantity of 2 drops into each nostril with following massage for spreading preparation in the mucosa. The procedure was repeated twice a day. The curative effect was controlled by general state of the organism and body temperature.

Example 19

The pharmacological preparation on the basis of a BAS, obtained as in Examples 1÷4; 7÷9; 11÷13 and 15÷17, was produced after lyophilic drying of the mentioned BAS in the quantity of 0,001 per cent by mass, with the rest being an excipient. The 10% saccharose solution was used as an excipient. The preparation was used for bee feeding. In comparison with control group, one feeding of a bee family in an amount of 1 liter of the preparation ensures, the following increase:

- survival by 85÷140%;
- quantity of flights by 100÷160%;
- honey by 27÷64%.

### Example 20

The pharmacological preparation on the basis of a BAS, obtained as in Examples 1÷6; 9÷14; 16÷21 and 23÷26, was produced after lyophilic drying of the mentioned BAS in the quantity of 85,0 per cent by mass, with the rest being an excipient.

The preservative was used as an excipient. After lyophilization a preparation in the form of powder was used for healing open wounds in case of damages, traumas, erosions, and burnings of the mucosa. The usage of preparation accelerated regenerative processes approximately by 2÷7 times in comparison with control group.

### Example 21

The pharmacological preparation on the basis of a BAS, obtained as in Examples 1÷4; 7÷9; 11÷13 and 15÷17, was produced in the quantity of 1,0 per cent by mass, with the rest being an excipient. The gel from plant pulp in the form cucumber-like, apple-like and peach-like puree was used as an excipient. The preparation was used for cosmetic purposes. The curative pharmacological effect controlled by cutaneous elasticity, was shown after 4÷8 seances.

### Example 22

The pharmacological preparation on the basis of a BAS, obtained as in Examples 1÷4; 7÷9; 11÷13 and 15÷17, was produced in the quantity of 1,0 per cent by mass, with the rest being an excipient. The gel based on honey (15 parts), onion juice (3 parts), garlic juice (1 part) and agrimony (burdock) oil (1 part) was used as an excipient. The preparation was used as a mask for strengthening hair-covering of the head. The head hair-covering was covered by a gel applications for 2÷3 hours. The procedure was repeated once a week. The curative pharmacological effect controlled by the state of head hair-covering was noted after 5÷12 seances.

### Example 23

The pharmacological preparation on the basis of a BAS, obtained as in Examples 1÷4; 7÷9; 11÷13 and 15÷17, was produced in the quantity of 1,0 per cent by mass, with the rest being an excipient. Lanoline mixed with spermaceti in the proportion of 1:3 was used as an excipient. The preparation in the form of an ointment was used for the regeneration and reconstruction of cutaneous covering after dermatosis. The usage of preparation induced 2÷5 fold increase of regenerative processes in comparison with control group.

### Example 24

The pharmacological preparation on the basis of a BAS, obtained as in Examples 1÷4; 7÷9; 11÷13 and 15÷17, was produced in the quantity of 1,0 per cent by mass, with the rest being an excipient. The cacao oil was used as an excipient. For the treatment of endometritis the preparation in the form of suppositories. The curative pharmacological effect controlled by the indices of inflammatory reaction, histological studies, blood analysis and some secretions was observed after 14÷21 days of usage.

### Example 25

The pharmacological preparation on the basis of a BAS, obtained as in Examples 1÷4; 7÷9; 11÷13 and 15÷17, was produced in the quantity of 1,0 per cent by mass, with the rest being an excipient. Lanoline mixed with vaseline in the proportion of 1:3 was used as an excipient. The preparation in the form of an ointment for was used treating mucous erosions and in the processes of their regeneration and reconstruction, a 3÷5 fold increase of its beneficial effect in comparison with control group was observed.

MATERIALS AND RESEARCH METHODS

### Example 26

The amino acid analysis of BAS compound, obtained as in Examples 1÷18, was processed in three directions, determining as follows:

- Total amino acid content,
- Amino acid content of peptides and free amino acids in absence of acid unsolvable proteins;

- Content of free amino acids.

1) The amino acid analysis of a BAS, obtained as in Examples 1÷18 was carried out by hydrolysis of the substance with sexinormal hydrochloric acid (6 N HCl) at a temperature of 110°C for 24 hours in vacuum. After the termination of hydrolysis the samples were evaporated on dry sodium hydroxide (NaOH in vacuum. The dry residue was diluted with didecimolar (0,2 N) sodium citrate buffer pH 2,2 and was put on ion-exchange columns of amino acid analyser.

2) For removing acid unsoluble proteins 0,5 ml of 3% sulphosalicylic acid were added into 0,5 ml of the sample and stayed for 1 hour, with periodic stirring. The denaturated proteins were removed by centrifugation at 8000 x g for 15÷20 minutes. The obtained supernatant was subjected to total amino acid analysis.

3) The free amino acid content of a BAS obtained as in Examples 1÷18, was determined following sample equilibration with didecinormal (0,2 N) sodium citrate buffer at pH 2,2 and after filtration through paper filter (with removed ashes) filtrate was put on the ion-exchange columns of amino acid analyser. The amino acid columns were eluted with sodium citrate buffer pH 3,25; pH 4,25 and pH 5,28.

The fractionated amino acids were treated with ninhydrin reagent and the optical density was determined by flow photometer at the wave length of 440 and 570 nm.

Example 27

The total lipid content of a BAS, obtained as in Examples 1÷18, was determined as follows. 0,5 ml of a BAS and 1,5 ml of concentrated sulphuric acid ($H_2SO_4$) were mixed and boiled at a temperature of 95°C for 15 minutes. After cooling, 1,5 ml phosphovanile reagent, (made by "Hemapol" firm, Czechoslovakia) was added to 0,1 ml hydrolyzate according to the well known method of determining total lipides by specific staining (J. M. Knight, S.Anderson, J.M.Rawle.- Clinical Chemistry -1972, -18,- p.199). The optical density of solution was determined at the wave length of 530 nm.

Example 28

The nucleotide content analysis of a BAS, observed as in Examples 1÷18, was carried out as follows.

The acid soluble purine and pyrimidine nucleotides were extracted from 1,5÷5,5 ml sample with 5% cooled chloric acid ($HClO_4$). In 20 minutes, after the extraction all cooled samples were centrifugated at 2500 x g for 10 minutes, the sediment was washed with 5% chloric acid ($HClO_4$) three times, the supernatant was collected and used for extraction of acidsoluble nucleotides.

The acid soluble fraction was hydrolyzed by monomolar chloric acid (1 M $HClO_4$) at a temperature of 100°C for 1 hour up to pyrimidine monophosphates and purine bases were obtained. The solutions were neutralized by potassium hydroxide (KOH) and all products of the hydrolysis were separated in a cation-exchanger "DAUEX" 50x4, using the step elution:

- with distilled water ($H_2O$) - for uric acid and uridinemonophosphate (UMPh);
- didecimolar chloric acid (0,2 M $HClO_4$) - for xanthine and cytosine monophosphate (CMPh);
- tetradecimolar chloric acid (0,4 M $HClO_4$) - for hypoxanthine;
- monomolar chloric acid (1,0 M $HClO_4$) - for guanine;
- dimolar chloric acid (2,0 M $HClO_4$) - for adenine.

Nucleotides were identified by the chromatographical activity in a kation-exchanger comparing with standards, and also by absorption spectrums at the wave length of 200÷300 nm. Nucleotide number was defined, using the coefficient of molar extinction.

Example 29

The immunomodulating activity (properties) of a BAS, obtained as in Examples 1÷18, was defined by macrophage activation of peritoneal exudate from Wistar rats in experiments in vitro. The task of these experiments was an increase of enzyme activity in the recovery of nitro-blue tetrazole into diphormazane (NBT-test) and an increase of phagocyte activity by E.Coli.

20 ml of the colourless Henk's solution, consisting of 20 units/ml of heparin, was injected into the abdominal cavity of decapitated animals by syringe.

In 2÷3 minutes after abdominal massage the incision was performed, and injected liquid was drained from cavity by the same syringe. Then, after filtration through nylon filter, this liquid was centrifugated at 150÷200 x g for 15 minutes.

The produced sediment was suspended with semivolumed fresh portion of the Henk's solution and recentrifugated under the same conditions.

The 3÷4 fold washing of extracted cells was processed, and after that cell concentration of the suspension was reached 80 x $10^6$ cells/ml. The macrophage content of tissue exudate consisted of 25÷35% in all cells.

The determination of macrophage, enzyme activity in rat peritoneal exudate was performed by spectrophotometry with the recovery of nitro-blue tetrazole in diphormazane. The cell sediment of rat peritoneal exudate was dissolved by the colourless Henk's solution up to the concentration of 80 x $10^6$ cell/ml.

50 mkl of the sample with pH 7,4 and peptide concentration of 1,0 mg/ml were added into 50 mkl of cell suspension.

This mixture was incubated at a temperature of 37°C for 30 minutes in water bath with permanent shaking. After that 50 mkl of the solution of nitro-blue tetrazole (made by "Reanal" firm (Hungary)), saturated at a temperature of 20°C, was added and incubation was continued for additional 30 minutes.

Then 3 ml of acetone were added into solution with following centrifugation at 2 000 x g for 20 minutes. The layer of acetone extract formed over sediment was subjected to photometry at the wave length of 515 nm.

The sample with a preservative based on physiological solution was used for comparing an enzyme activity.

The phagocyte activity of rat peritoneal exudate cells was defined by their ability to E.Coli capture.

50 mkl of the sample with pH 7,4 and peptide concentration of 0,05 mg/ml was added to 50 mkl of cell suspension.

This mixture was incubated at a temperature of 37°C for 30 minutes in water bath with permanent shaking. Finishing incubation the mixture was suspended with 10 ml of the colourless Henk's solution at a temperature of 4°C, with subsequent centrifugating at 150÷200 x g for 15 minutes.

The cell sediment of rat peritoneal exudate was diluted by Henk's solution up to the concentration of 2,5 x $10^6$ cells/ml. The produced suspension in an amount of 0,2 ml was put on the glass sized 18 x 18 mm and incubated at a temperature of 37°C for 30 minutes in the atmosphere, comprising 5% carbon dioxide ($CO_2$) for fixing macrophages on glass.

After the incubation, in order to remove some non-fixed cells, the glass was washed in the tumbler with Henk's medium 3 times.

0,2 ml of E.Coli suspension with concentration of 20 x $10^6$ cells/ml were added to remained cells on the glass and then incubated under the similar conditions for 45 minutes. Then a glass was rinsed three times, fixed with methanol and coloured by Romanovsky - Gymze.

The index of phagocytosis (IPh) was defined by the following formula :

$$IPh = (0 + 2,5n + 6t + 9p + 10R)/ \text{cells number,} \qquad (1)$$

where:

0 - cell number, which does not phagocytize E.Coli;
n - cell number, which absorbed 1÷4 cells;
t - cell number, which absorbed 5÷7 cells;
p - cell number, which absorbed 8÷9 cells;
R - cell number, which absorbed 10 and more E.Coli cells.

The identification of macrophages was processed by colouration of unspecific esterase.

<u>Example 30</u>

The immunomodulating activity (properties) of a BAS observed as in Examples 1÷18, was defined by activation of Wistar rat blood neutrophiles in experiments in vitro. The task of these experiments was increasing of enzyme activity of nitro-blue tetrazole recovery into diphormazane (NBT-test) and the increase of phagocyte activity by E.Coli.

The blood from rat tail artery was collected into test-tube with the colourless Henk's solution on heparin and mixed with periodic stirring.

6% dextran T-500 in proportion of 3:1 was added to obtained mixture and was placed in the refrigerator for one hour. After erythrocyte sedimentation the leucocyte layer formed over sediment was collected by pipette and centrifugated at 250 x g for 15 minutes. In order to remove erythrocytes from leucocytic mass the obtained substance was hemolyzed by suspending leucocyte sediment in distilled water with adding Henk's solution and mixture was centrifugated once more. The sediment of leucocytes was resuspended with fresh Henk's solution and centrifugated under the same conditions. The operation of washing was repeated 3 times.

The definition of enzyme activity of blood neutrophiles was performed by the spectrophotometer with the reduction of nitro-blue tetrazole into diphormazane.

The sediment of rat blood leucocytes was diluted by the colourless Henk's solution up to the concentration of 80 x $10^6$ cells/ml.

100 mkl of the cell suspension was added to 300 mkl of a BAS, received as in Examples 1÷18, with adjsting up to pH 7,4 and peptide concentration up to 1,0 mg/ml.

This mixture was incubated at a temperature of 37°C for 30 minutes in water bath with permanent shaking. After that 400 mkl nitro-blue tetrazole solution (made by "Reanal" firm (Hungary)) was added, and all operations were carried out as in Example 29.

The phagocyte activity of leuocytes in rat blood cells was defined by their ability to E.Coli capture.

50 mkl of leucocyte suspension was added to 50 mkl of a BAS with pH 7,4 and peptide concentration up to 0,05 mg/ml.

This mixture was incubated and, then, 10 ml colourless Henk's solution at a temperature of 4°C was added with subsequent centrifugation at 150÷200 x g for 15 minutes.

The cell sediment was diluted by 0,3 ml of Henk's solution and added with 0,05 ml E.Coli at the suspension concentration of 500 x $10^6$ cells/ml. The produced suspension was incubated at a temperature of 37°C for 30 minutes. After the incubation some smears were done, and after colouring the quantity of phagocyting cells and phagocytosis index (IPh) were measured as in Example 29.

Example 31

The immunomodulating activity (properties) of a BAS, obtained as in Examples 1÷18, was defined in experiments on BALB/C mice in vivo. The animals were received 2 subcutaneous injections of preparation at a dose of 0,5 ml/kg of the body weight with two day intervals between injections at peptide concentration of 1,0 mg/ml. The analysis of immunomodulating properties was performed in two hours after last injection. No less than 10 animals were used in each experiment. The animals, treated with 0,08% preservative diluted in physiological solution, were used as control group.

For determining the reaction of lymphocyte blast-trasformation, phitohemaglutenin (PhGA) was used, as T-cell mitogene and, lypopolysaccharid E.Coli (LPS) was used as B-cell mitogene.

The lymphocytes, extracted from rat spleen, were incubated with these mitogenes at a temperature of 37°C for 72 hours in the atmosphere comprising 5% carbon dioxide ($CO_2$). The level of cell proliferation was defined radiometrically by the index including $^3$H-hymidin. In order to do it $^3$H-hymidin (3,7 x $10^4$ Bk/probe) was added to cultures and incubated for 3 hours. Then the samples were put on the millipore filter ("Synpor" N5, Czechoslovakia) with subsequent 3 fold washing including medium 199, 5% solution of trichloroacetic acid (TChA) and ethanol. The filters were dried, put into the bottles with scintillation liquid (LS-106) and the radioactivity of the samples was defined by "Mark-111" counter.

Example 32

The immunomodulating activity (properties) of a BAS, received as in Examples 1÷18, was defined by the stimulation of natural killer activity in mouse splenocytes in experiments in vivo. The animals were received 2 subcutaneous injections of preparation at a dose of 0,05 ml/kg of the body weight for 2 days at peptide concentration of 1,0 mg/ml. The analysis of immunomodulating properties was performed next day after last injection. No less than 10 animals were used in each experiment. The animals, treated with 0,08% preservative diluted in physiological solution, were used as control group.

The state of natural killer activity in splenocytes was defined on the YAC-12 mouse lymphoma cells labelled by chrome isotope $^{51}$Cr. The incubation was performed for 18 hours with correlation between target-cells and effector-cells (1 x $10^5$: 2,5 x $10^6$ cells) in the proportion of 1:25. The specific release of chrome isotope $^{51}$Cr, corresponding to cytostatic activity of cell-effectors, was calculated by the following formula:

$$^{51}Cr = \frac{(\text{release in an experiment}) - (\text{self-release})}{(\text{ maximum release }) - (\text{ self-release })} \times 100, \qquad (2)$$

where: "maximum release" means - radioactivity, and under such conditions all cells are lysed. The preservative, diluted in physiological solution was used as control for the definition of increased natural killer activity induced by the proposed BASes.

Example 33

The immunomodulating activity of a BAS obtained as in Examples 1÷18, was defined by the functional activity of mouse thymus in experiments in vivo under analogical conditions described in Example 31.

The effect was estimated by titre of thymic serum factor (TSF). This method is based on the ability of thymic hormones to restore the sensitivity of spontaneous rosette-forming cells from the spleen of mature mice, to antithymocytic serum, after thymectomy.

The blood serum of experimental animals was ultrafiltrated through the filter of the system CENTRIFLO CF-50A (made by "Amicon" company). 0,1 ml of the undiluted and serially diluted by the medium 199 serum, was used for the reaction. Medium 199 was used as a control.

The mouse spleens were obtained in 10÷14 days after thymectomy. The spleen cells were extracted by the method of tissue defibrination with preparative needles in the medium 199. After the filtration through caprone sieve, the cells were twice washed with the medium 199 by centrifugation at 4 000 x g. The sediment was resuspended in the medium up to the concentration of $4 \times 10^7$ cells/ml and equilibrium obtained in the volume 0,1 ml, was added to the test - tubes containing serum and medium 199. The consistence of the test - glass was mixed by pipette and incubated at a temperature of 37°C for 60 minutes.

After the incubation 0,1 ml of antithymocyte serum and a complement of the guinea-pig in dilution 1:10, were added to all test-tubes. The suspension was incubated at a temperature of 37°C for additional 30 minutes. Then 0,1 ml of the suspension of rat erythrocytes ($12 \times 10^7$ cells/ml) was added, mixed and centrifugated at 250 x g for 5 minutes with subsequent incubation at a temperature of 4÷8°C for 60 minutes. The sediment of test - tubes was resuspended for 5 minutes. The rosette calculation was performed in Goriajev's camera. The lymphocyte, binding four and more erythrocytes was considered as a rosette. The last dilution of serum, inducing 50% reduction of the number of rosette-forming cells (RFC) responding to the control was considered as Titre TSF. The results were expressed in the form of logarithm of titre with the basement 2, namely in the form of log A, where A is a titre meaning.

Example 34

The efectiveness of a BAS, produced as in Examples 1÷18, manifested in the increase of reparative abilities of rat liver hepatocytes in vivo after subcutaneous injection of carbon tetrachloride ($CCl_4$), was defined by the rehabilitative and reconstructive indices of Na,K-ATPase activity as follows.

Everyday Wistar mature male rats weighting 230-250 g were received subcutaneous injection of carbon tetrachloride ($CCl_4$) mixed with the vegetable oil in proportion of 1:1 at a dosage of 4,0 ml/kg of the body weight. In 4 hours after the first injection of carbon tetrachloride ($CCl_4$), the experimental animals were received the first injection of preparation at a dose of 0,05 ml/kg of the body weight with one day interval between injections. The efficiency of a substance was registered the next day after last injection. As a control animal liver, analogically damaged by carbon tetrachloride ($CCl_4$), as well as the liver of intact animals untreated with the studied BAS, were used for comparison.

In order to determine the Na, K-ATPase activity, fractions of hepatocyte cell membranes were used. In order to do it, 15 g of cooled liver of three rats were homogenized in 150 g of millimolar borate buffer solution (1MM $Na_2B_4O_7$) with pH=7,5, and in the presence of quinquedecimillimolar solution of calcium chloride (0,5 MM $CaCl_2$), using the hand glass-teflon homogenizer.

The homogenized mixture was processed by the method of centrifugation at 150 x g at a temperature of 4°C for 10÷12 minutes. Then, the supernatant was recentrifugated at 100 000 x g for 20 minutes, and obtained sediment was three times washed in 130 ml of buffer solution under the similar conditions of centrifugation. After finishing washing the sediment was resuspended in buffer solution, with protein concentration adjusted (according to the method of SDS-Louri) up to 3,0 mg/ml. The obtained suspension was used in the subsequent analysis.

All operations of the production of membrane fraction were processed under the cold conditions.

The Na, K-ATPase activity of hepatocyte plasmatic membranes was defined in the standard medium, comprising:

sixty six millimolar sodium chloride (66 MM NaCl);
thirty four millimolar potassium chloride (34 MM KCl);
quinquemillimolar magnesium chloride (5 MM $MgCl_2$);
twenty five millimolar (25 MM) Tris-HCl, with subsequent adding quinquemillimolar adenosine triphosphoric acid (5 MM ATPh) before the test.

0,2 ml of the suspension of membrane fraction with protein concentration of 3,0 mg/ml was added to 1,8 ml of the mentioned standard medium and was incubated at a temperature of 37°C for 15 minutes. After that the reaction was stopped and 0,6 ml of 50% solution of trichloroacetic acid (TChA) was added. The mixture was centrifugated at

300÷500 x g for 15 minutes. To 1,0 ml of the obtained supernatant the following stuff was added: 4 ml of didecimolar (0,2 M) acetate buffer solution with pH=4,0; 0,5 ml of 1% solution of ammonium molibdate in 1% solution of sulphuric acid ($H_2SO_4$); 0,5 ml of ascorbic acid in 0,16% solution of ($CuSO_4$).

After mixing all this compounds were incubated at a temperature of 25°C for 10 minutes. The optical density was defined spectrometrically at the wave length of 700 nm.

The activity of Na,K-ATPase was defined by the difference between the activity of general ATPase and Mg-ATPase. In order to define the activity of Mg-ATPase there were processed the similar performances, but 0,3 ml of 0,05% solution of strophanthin instead of 0,6 ml of trichloracetic acid (TChA), were added.

It was established that in the toxical damage of liver induced by carbon tetrachloride ($CCl_4$) 3,5 fold decrease in Na, K-ATPase activity was observed while using the declared BAS, as in Example 11, its activity can be recovered experimentally no less than by 50% in comparison with the norm. Under the same conditions the well known hepatotropic preparation "Essentiale" - an industrial analogue of the declared preparate, reconstructes Na, K-ATPase activity by 43,0±4,8%.

Example 35

The effectiveness of a BAS, produced as in Examples 1÷18, that manifested in the increase of reparative abilities of rat liver hepatocytes (in vivo) was defined after the subcutaneous injection of carbon tetrachloride ($CCl_4$), according indices of aminotraspherase activity in blood and potassium (K) concentration in hepatocytes of the rat liver as follows.

The aminotraspherase activity of animal blood, processed as in Example 34, was tested on the test-system of "Hemapol" company (Czechoslovakia) according to the photometrical method with the specific colouring (S.Reitman, S.Frankel.- American Journal of Clinical Pathology,-1957-28-56).

0,05 ml of the serum of rat blood were added to 0,25 ml of the solution, consisting of the following components: decimolar solution of (0,1M) L-aspartate; didecimolar (0,002 M) 2-oxoglutarate; and decimolar (0,1 M) phosphate buffer solution with pH=7,4. This mixture was incubated at a temperature of 37°C for 60 minutes. After finishing incubation 0,25 ml of the solution consisting of millimolar (0,001M) 2,4-dinitrophenilhydrazine, diluted in monomolar hydrochloric acid (1 M HCl), was added into the solution. The sample was left for 20 minutes at a room temperature. After that 2,5 ml of tetradecimolar (0,4 M) dry sodium hydroxide (NaOH) was added, mixed and, after 10 minutes, the optical density was measured, at the wave length of 520 nm comparing with the control solution, where 0,05 ml of physiological solution instead of 0,05 ml of blood serum were added.

It was established that in toxical damage of the liver induced by carbon tetrachloride ($CCl_4$), hepatocyte destruction is occured, and, correspondingly, the release of aminotranspherases, with the consequent 2 fold increase of their activity in blood serum is observed.

At the same time the usage of a declared BAS, obtained as in Example 11, allows to restore such activity no less than by 59% experimentally in comparison with the norm.

Under the same conditions the well-known hepatotropic preparation "Essentiale" reduced the aminotranspherase activity in blood by 87,2÷7,4%.

The potassium ($K^+$) concentration in hepatocytes of the liver of experimental rats was defined by the standard method of flame photometry, according to Brikker, using the flame photometer, type FPhM-1 (V.N.Brikker, The definition of K, Na content by the method of the flame photometry. - Laboratornoe delo-1961-7- p.3-6).

It was established, that in the case of toxical damage of liver induced by carbon tetrachloride ($CCl_4$) Na, K-ATPase activity was decreased by 3,5 times, with following decrease in $K^+$ concentration of rat liver hepatocytes by 40%. In the same time the usage of a declared BAS, obtained as in Example 11, affords the reconstruction of $K^+$ concentration no less than by 40%. The well known preparation "Essentiale" reduced K+ concentration of hepatocytes by 33,0+6,2% under the similar conditions.

The invention is not framed to explain concrete variants of its executing. The different variants can be carried out in the ranges of essence and scope, covered by the formula of the invention.

ANALYSIS OF THE COMPOSITION AND PROPERTIES OF THE DECLARED BAS AND THE PREPARATION MADE ON ITS BASIS

The identification of a declared BAS among other biologically active substances can be made directly by biochemical assay and indirectly - by a size of immunomodulating effect which is displayed by the declared substance.

It was established, that just modified components of cell membranes with modified antigenic structure ensure a considerable increase in immune reaction of the organism in comparison with control. This allows a declared BAS to be identified by the following set of indices.

a) An increase in macrophage activity of rat peritoneal exudate, particularly in enzyme activity of macrophages, controlled by NBT-test, no less than by 54% (in the case of nitro-blue tetrazole (Hungarian company "Reanal") use in experiments) an increase in phagocyte activity of E.Coli - no less than by 39%;

b) An increase in activation of rat blood neutrophils, namely an increase in enzyme activity of neutrophils, no less than by 67%. controlled by NBT-test, (in case of nitro-blue tetrazole (Hungarian company "Reanal") use in experiments); an increase in phagocyte activity of E.Coli - no less than by 44%;

c) An increase in the reaction of blasttransformation of T- lymphocytes no less than by 42%, and B-lymphocytes no less than by 50%;

d) An increase in functional activity of the thymus - according to a content of the thymic serum factor (TSF) in rat blood no less than by 167%;

e) An increase in natural killer activity of rat splenocytes no less than by 66%;

f) An increase in ability to stabilize plasmatic membranes of rat liver hepatocytes after injection of carbon tetrachloride ($CCl_4$):

- recovery of Na, K-ATPase activity no less than by 24%;
- recovery of transpherase activity in rat blood no less than by 22%;
- normalization of potassium (K) to sodium (Na) ratio in rat liver hepatocytes no less than by 13,8%;

The preservative diluted with physiological solution was used as a base for comparison.

This index system of immunomodulating and regenerating properties of the declared BAS allows its identification by biological activity as an immunomodulator and stimulator of regenerative processes.

For the declared BAS and preparation on its basis there was notified a tendency to display the stimulation of protective functions of the immune system of an organism, directed as well as to elimination of internal pathology (stimulation of natural killer activity), and an increase in general resistance of an organism to infectious diseases, that considerably exceeds the efficiency of analogical indices of the above mentioned well known BASes.

The immunomodulating properties of various biologically active substances accordingly to complex of indices, identificating researched objects were determined experimentally. The results of experiments are summarized in Table 6, where the quantitative indices of effects of the well known BASes No1, No2, No3, obtained according the technology, described in EP-A-02499563, SU-A-139404 and US-A-4455302 correspondingly as well as the declared BAS, represented in the form of groups WS, TS and LM, namely, generalized including the results are shown:

WS - Examples 1÷4 (components of morphoplasm and glycocalyx of cells);
TS - Examples 7÷9 (thermostable components of morphoplasm and glycocalyx of cells);
LM - Examples 11÷13 (low-molecular components of morphoplasm and glycocalyx of cells M≤10,0 kDa).

The physiological solution of sodium chloride with a preservative was used as control. The results obtained allowing comparison between the declared and known BASes are summarized in Table 6.

These results are dealt with the characteristics of a declared BAS produced in the form of a mixture with undetermined structure. The findings which are necessary for its identification are also represented.

At the same time the analysis of Table 6 affords to identify declared substance and appreciate the concrete result, ensured by the declared BAS that is concerned in essential improvement of the properties of biological activity. It can be achieved owing to the presence of some hydrolysis products in it which consist of some modified components of morphoplasm and glycocalyx of cells with the modified structure, and their influence in the well known BASes is not revealed.

The properties of a declared BAS and preparation on its basis directly depend upon its content.

According to biochemical analysis (Table 7), the BAS

Table 6

| Immunomodulating and regenerating effects of biologically active substances (BASes) | | Quantitative indices of BAS effects | | | | | |
|---|---|---|---|---|---|---|---|
| | | Well known | | | Declared BAS | | |
| | | N 1 | N 2 | N 3 | WS | TS | LM |
| 1. An increase in the activity of macrophages of rat peritoneal exudate versus control, % | Enzyme activity (NBT-test) | 27,1±2,6 | 42,2±2,4 | 46,8±4,3 | 62,6±8,4 | 87,8±4,8 | 142,6±15,6 |
| | Phagocyte activity by E.Coli | 15,6±2,1 | 21,4±4,2 | 33,4±4,3 | 46,8±7,7 | 64,3±9,6 | 120,4±4,3 |
| 2.An increase in the activity of blood neutro phyles versus control, % | Enzyme activity (NBT-test) | 33,8±15,9 | 60,6±3,7 | 67,2±13,8 | 76,2±8,8 | 95,4±13,8 | 158,9±20,6 |
| | Phagocyte activity by E.Coli | 25,4±1,6 | 34,6±3,7 | 44,2±5,7 | 52,3±7,5 | 70,4±8,5 | 123,6±10,7 |
| 3. Stimulation of blasttransformation reaction versus control , % | T-lymphocytes | 24,6±2,8 | 25,1±3,7 | 38,4±4,6 | 46,6±4,7 | 69,5±10,2 | 134,8±14,5 |
| | B-lymphocytes | 22,1±3,2 | 28,8±2,4 | 46,3±4,7 | 55,6±5,4 | 82,6±10,7 | 168,2±21,4 |
| 4. An increase in titre of thymic serum-like factor(TSF) versus control, % | | 52,4±4, 2 | 56,7±6,2 | 117,8±9,1 | 208,6±41,2 | 318,7±45,6 | 624,7±81,3 |
| 5. Stimulation of natural killer activity of rat splenocytes versus control,% | | 65,6±4,8 | 17,6±4,2 | 53,8±5,4 | 72,3±6,2 | 105,3±15,3 | 194,7±16,3 |
| 6. An increase in reparative abilities of rat hepatocytes by reconstruction of activity after $CCl_4$ injection[4] versus control,% | Na, K-ATPases | 0 | 13,2±3,1 | 22,6±3,4 | 26,7±2,4 | 31,4±3,2 | 57,6±7,2 |
| | Blood aminotranspherases | 0 | 0 | 23,4±4,8 | 27,2±5,1 | 46,2±7,4 | 70,6±11,4 |
| | $K^+$ Concentration of hepatocytes | 0 | 5,2±1,2 | 14,2±3,1 | 16,8±3,0 | 24,6±2,4 | 48,2±4,3 |

Differences are significant with control P<0,05

Table 7

| Organic compounds | Content of organic compounds mass.% | | | | | |
|---|---|---|---|---|---|---|
| | Known BASes | | | Declared BAS | | |
| | N 1 | N 2 | N 3 | WS | TS | LM |
| Polypeptides | 72,7 ±9,4 | 47,4 ±8,7 | 32,1 ±6,1 | 20,0 ±3,5 | 9,1 ±3,6 | than 0,3 |
| Peptides | 13,5 ±2,1 | 35,8 ±7,4 | 16,3 ±3,2 | 15,0 ±5,0 | 17,3 ±3,7 | 18,7±3,8 |
| Amino acids | 4,7 +1,7 | 3,7 +0,8 | 31,7 +6,2 | 40,1 +8,1 | 45,0 17,9 | 51,8±8,3 |
| TOTAL: | 90,9 ±6,0 | 86,9 ±5,8 | 80,1 ±5,1 | 75,1 ±5,1 | 71,4 ±4,8 | 70,5±5,3 |
| Content of carbohydrates - high-molecular compounds with a molecular mass M>10,0 kDa | 0,5 ±0,2 | 0,9 ±0,2 | 1,9 ±0,8 | 0,7 ±0,4 | 0,4 ±0,2 | than 0,2 |
| -low-molecular compounds with a molecular mass M=0,8÷10,0 kDa | than 0,2 | 2,7 ±1,1 | 3,0 ±0,5 | 6,8 ±3,5 | 7,5 ±3,5 | 8,2 ±3,3 |
| -free monomers or compounds with a molecular mass M<0,8 kDa | 7,3 ±2,4 | 2,1 ±0,6 | 10,2 ±2,7 | 10,0 ±2,4 | 12,7 ±2,9 | 13,7±3,5 |
| IN ALL: | 8,0 ±1,8 | 5,7 ±1,6 | 14,9 ±2,7 | 17,7 ±3,1 | 20,6 ±4,5 | 22,1 ±6,6 |
| Lipides,nucleotides and other organic compounds | 1,1 ±0,3 | 7,4 ±2,1 | 5,0 ±1,6 | 7,2 ±2,1 | 8,0 ±2,7 | 7,1±2,1 |
| TOTAL: | 100,0 | 100,0 | 100,0 | 100,0 | 100,0 | 100,0 |
| Correlation between carbohydrate and peptide contents G* | than 0,01 | 0,08 | 0,18 | 0,45 | 0,43 | 0,44 |
| Weight of dry residue of BAS mg/ml | 20,0 ±4,2 | 11,4 ±3,7 | 32,6 ±9,6 | 12,8 ±3,6 | 8,4 ±2,2 | 6,1 ±1,8 |
| Weight of preservative mg/ml | - | - | - | 0,7 | 0,7 | 0,7 |
| BAS concentration in powder (composition) mass % | - | - | - | 94,8 ±1,1 | 92,3 ±1,5 | 89,7±2,2 |

Differences are significant with control P<0,05
* Values are the mean of determinations as indicated ± SEM

includes the following organic compounds such as: polypeptides, peptides, amino acids, bound and free carbohydrates, lipides, nucleotides and others in its content. Table 7 shows the distribution of organic compounds in BAS content by mass during usage of embryonal tissue of horned cattle as initial raw material. Besides, the results of obtaining various groups of the declared BAS (WS, TS and LM) are shown in Table 7 in strict succession of all declared methods.

Besides the results of processing, which ensures obtaining of BAS-WS when as the raw material, animal tissue taken on the stage of the process of modification of components of morphoplasm and glycocalyx of cells with the above described processes was used Table 7 includes also the results of obtaining a declared BAS, in the form of thermostable components of BAS-ST, as well as in the form of low-molecular components of BAS-LM.

The data of Tables 6 and 7 affords to make the comparative analysis of the results of realization of the declared and well known methods of obtaining a BAS, taking into consideration the fact, that BAS N3 (US-A-4455302), BAS N2 (SU-A-139404) and BAS N1 (EP-A-0249563) among the well known ones are the best by their characteristics.

The comparative analysis of specific activity of the declared BAS and well known ones (Tables 6 and 7) shows, that their specific activity depends upon content of ingredients, and their proportion has a considerably influence on the quantitative index of specific activity.

The characteristic feature of biochemical content of a declared BAS is the presence of some carbohydrates comprising components, which modification in high degree defines its effect. At the same time carbohydrate content of the most active fractions (fractions 3, 4 and 5 in Table 4) of low-molecular components with a molecular mass M=0,8÷10,0 kDa (oligocarbohydrates, glycopeptides, glycolipides, nucleotides) is the decisive factor in identification and characterization of the declared BAS.

Thus, the comparative analysis of specific activity and ingredient content (Tables 6 and 7) shows, that the higher activity of the declared BAS, correlates with higher carbohydrate content in components with a molecular mass M=0,8÷10,0 KDa in comparison with analogues. For the declared substance these significances are equal to the following values: 6,8±3,5% ; 7,5±3,5% and 8,2±3,3% , while for analogues - BAS No 1, No 2, No 3 they are: no more than 0,2%; 2,7±1,1% and 3,0±0,5%, correspondingly. This dependence correlates with a specific content of carbohydrates in respect to the other organic compounds of this fraction to an even greater degree.

In this respect the essential index is the proportion of carbohydrate and peptide contents in components with a molecular mass M=0,8÷10,0 kDa (Tables 2 and 7), which can be determined in accordance with the formula 3:

$$G = \frac{Cc}{Cp} \qquad (3),$$

where Cc и Cp - are concentrations of carbohydrates and peptides in components with a molecular mass M=0,8÷10,0 kDa.

Index value of the declared BAS lays within the limits Gd=0,25÷0,60 while value of this index for well known BAS does not exceed Gwk<0,25.

So, properties of the hydrolysis products were characterizes by value of index G and depend upon raw material used in declared method, upon total combination of operations of processing a mentioned raw material and the regimes of realization of hydrolysis processes, and it was one of characteristic indices of biochemical identification of the declared BAS.

The whole complex of indications of a declared biologically active substance and totality of the indications of a declared method of its production and declared pharmacological preparation on the basis of this substance, are connected with the specific activity of the BAS as cause and effect.

THE INDUSTRIAL USE

The inventions related to a BAS, a method of its production and a pharmacological preparation on the basis of the declared BAS, are based on the selection of initial raw materials, the choice of conditions for processing and obtaining a BAS and a preparation with guaranteed high specific activity. The chosen technology for obtaining a BAS ensures the reproductivity of results, that affords to standardize obtaining of the declared BAS with desired properties, namely, with given fixed immunomodulating activity. This activity defines the pharmacological, therapeutical and generally biological effects in cases of this invention usage.

According to character of their biological influence, the obtained desired products in general and particular cases realizing the declared method for obtaining the BAS are equal, but they are different by their specific activity. Thus the advisable use of this or another concrete biologically active substance is determined by the requirements of medicine and/or veterinary. In this connection, the selection of a certain BAS from others is determined by the correlation between demands to its immunomodulating activity and the effectiveness and the cost (economy) of its production in every specific case of usage of the desired product. Particularly the high efficiency of substance, comprising low-molecular components of the declared BAS-LM, with a molecular mass M≤10,0 kDa is completely displayed in the form of increased activity of macrophages, neutrophiles, lymphocytes, natural killers, that ensures the strengthening of the regenerative processes (Table 6). For agricultural animals such effects are followed by additional increase of animal weight in the case of intensive fattening (Table 8). In the treatment of respiratory and infectious diseases (parainfluenza) such an advantage is not significant and so the use of a BAS, comprising water-soluble components of morphoplasm and glycocalyx of the cells of both BAS-WS and BAS-PM will be more economical. A substance, comprising thermostable components of the declared BAS-TS should be used in the treatment of inflammatory processes (mastitis, endometritis and so on).

Preparing preparations for external use in the form of ointments, gels, compresses, suppositories, intranasal drops, inhalations or animal drinks as wet feedings the use of a BAS, comprising thermostable components of BAS-ST instead of a BAS-LM will be more economical.

In order to receive the maximum effect it would be more reasonable to use the declared BAS for the activation and stimulation of protective functions of the immune system of an organism, directed both to elimination of the internal pathology (stimulation of natural killer activity) and to an increase in general resistence of an organism to infectious diseases, which in considerable degree exceeds analogical indices in the above described known BASes.

In addition, the declared BAS and preparations on its basis are more effective substances in the treatment

EP 0 673 652 B1

of infectious diseases (parainfluenza, ARD, and so on). Their using induce recovery of younger animals of horned cattle in 82÷93% cases, while the known BAS as well as antibiotics and a preparation "Boviverex", induce the recovery of animals no more, than 70% cases, and in the spontaneous recovery of control group - within the limits of 58÷67% cases.

The declared BAS increases the resistence of animals to infectious diseases, that considerably reduces the mortality level of the cattle population.

The utilization of this BAS as a prophylactic preparation reduced calfe morbidity associated with parainfluenza up to 10% from the total number, while the application of the well known BAS reduced the morbidity of animals up to 12÷17%, and in control group this index was reduced up to 22÷28%.

The normalization of general biological state of agricultural animals utilizing the declared BAS improves productive indices, particularly rises an additional increase in living weight, without lowering meat quality and simultaneously nearing these indices to genetically programmed level in the case of intensive fattening. This feature differs the declared BAS and preparations on its basis from hormonal and other growth stimulators, rising quantitative indices, with negative influence on animal homeostasis and reducing quality of the products.

In the case of intensive fattening, the administration of a declared BAS and preparations on its basis into animal organisms, with the declination of homeostasis parameters from the norm, ensures the rising of additional weight increase on average by 37÷100% comparing with control group without the use of a declared BAS, while using well known BAS analogues (of the substance No 1) induced additional increase of weight in comparison with the average by 12÷24% of control group under the similar conditions. The control and experimental groups of horned cattle consisted of 14÷18 calves of the same age, initial weight and etiology with declination from the norm.

The normalization of homeostatic parameters promotes an increase in:

- milk yields of the horned cattle, and small cattle;
- eggs laying of the poultry;
- fur quality of the fur-bearing animals;
- survival of younger animals;
- honey of bees and so on.

The declared BAS and a preparation on its basis are non-toxical, did not cause the side effects (allergies and so on).

The declared preparation causes increased stimulation of regenerative processes, namely, rehabilitation of activity of liver tissue in case of hepatitis and liver regeneration after partial hepatectomy, healing of ulcers, wounds and cutaneous surface in case of the damages and wounds, reconstruction of blood formula in case of great blood loss and in the other cases.

The comparative data of pharmacological, therapeutical and general biological effects of utilizing the declared BAS and a preparation on its basis in animals are shown in Table 8. The analysis of data in this table illustrates, that a biological activity of the declared BAS considerably exceeds that of analogues.

Thus, the declared BAS and a preparation on its basis, have a wide spectrum of abilities for their use in veterinary and medicine and can be used for the normalization of physiological state of the organism in treatment of diseases, controlled by the immune system.

According to the invention, a declared substance and preparation on its basis are used as follows. The parameters of homeostasis, are determined and in case of their declination from the norm, the stimulation of the immune system until normalization of pointed homeostasis parameters by BAS injection is performed.

33

Table 8

| Pharmaceutical, therapeutical and general biological effects of BAS | | | Quantitative characteristics | | | | | |
|---|---|---|---|---|---|---|---|---|
| | | | Known BASes | | | Declared BAS | | |
| | | | N 1 | N 2 | N 3 | WS | ST | LM |
| 1. Inflammatory processes | Mastitis of cows, recovery % | Exp. | 56±8 | 66±6* | 70±5 | 76±3 | 83±4 | 88±5 |
| | | Control | 59±7 | 57±6 | 61±5 | 58±8 | 63±7 | 53±8 |
| | Pneumonia recovery % | Exp. | 55±8 | 64±7* | 69±6 | 78±5 | 89±6 | 91±5 |
| | | Control | 58±7 | 60±7 | 55±7 | 51±8 | 61±6 | 48±6 |
| 2. Infectious diseases | Para-in fluenza & ARD*, recovery % | Exp. | 55±5* | 65±7 | 70±6 | 82±6 | 90±7 | 93±5 |
| | | Control | 60±5 | 62±6 | 64±7* | 58±6 | 61±7 | 67±5 |
| 3. Additional increase in calf weight versus control, % | | | -12±* 10 | 15±6 | 24±7 | 37±7 | 58±8 | 102±15 |
| 4. Acute toxicity LD$_{50}$ ml/kg (mg/kg) of body weight | | | more than 25 (260) | | | more than 25 (260) | | |

Differences are significant with control P<0,05 * P>0,05 * ARD - acute respiratory disease

The mentioned stimulation of the immune system is realized by the activation of reticulo-endothelial system, T- and B-systems, neutrophiles and/or popupation of natural killers of organism, that ensures the rise of macrophage activity of peritoneal exudate and blood neutrophiles in vitro, the rise of stimulation of blasttransformation reaction of lymphocytes in vivo, the rise of stimulation of splenocyte natural killer activity in vivo, as well as increase of reparative properties of liver hepatocytes after injection of poisoning agents or partial hepatectomy and so on.

For this purpose one can choose following methods of administration of the declared BAS and preparation on its basis:

- intramuscular injections at a dose of 0,005÷0,5 ml/kg of the body weight with 1÷7 day intervals between injections;
- inhalations at a dose of 0,012÷0,12 ml/kg of the body weight with 4÷48 hour intervals between inhalations;
- sublingual administration at a dose of 0,012÷0,12 ml/kg of the body weight with 4÷48 hour intervals between injections;
- wet feedings at a dose of 0,02÷1,0 ml/kg of the body weight with 1÷10 day intervals between wet feedings;
- enteral administration in the form of pills at a dose of 0,02÷1,0 ml/kg of the body weight with 1÷10 day intervals;
- intranasal administration at a daily dose of 0,001÷0,05 ml/kg of the body weight with 2÷24 hour intervals between administrations;
- applications to mucous or wound surface in the form of compresses, suppositories, powders, ointments, and gels;
- and their combinations, until homeostasis parameters will be normalized;

The data confirming an efficiency of used method of a declared BAS in the form of thermostable components of BAS-ST and preparation on its basis, in which the physiological solution of sodium chloride was used as an excipient, are represented in Tables 9-12. Tables show a dependence of

Table 9

| Effectiveness of intramuscular injections | | | | | |
|---|---|---|---|---|---|
| Dosage, mg/kg of body weight | less 0,005 (0,001) | 0,005 | 0,05 | 0,5 | more than 0,5 (1,0) |
| Weight increase of animals versus controls, % | 32 ± 2 | 47 ± 2 | 55 ± 5 | 61 ± 5 | 42 ± 3 |

Differences are significant with control P<0,05

Table 10

| Effectiveness of inhalations | | | | | |
|---|---|---|---|---|---|
| Dosage, mg/kg of body weight | 0,001 | 0,012 | 0,05 | 0,12 | 0,6 |
| Weight increase of animals versus controls, % | $10 \pm 2$ | $22 \pm 2$ | $25 \pm 3$ | $30 \pm 4$ | $20 \pm 2$ |

Differences are significant with control P<0,05

Table 11

| Effectiveness of wet feedings | | | | | |
|---|---|---|---|---|---|
| Dosage, mg/kg of body weight | 0,001 | 0,01 | 0,05 | 0,5 | 2,0 |
| Weight increase of animals versus controls, % | $10 \pm 4$ | $32 \pm 3$ | $35 \pm 3$ | $42 \pm 3$ | $30 \pm 4$ |

Differences are significant with control P<0,05

Table 12

| Effectiveness of intranasal administration | | | | | |
|---|---|---|---|---|---|
| Dosage, mg/kg of body weight | 0,0005 | 0,001 | 0,005 | 0,05 | 5,0 |
| Weight increase of animals versus controls, % | $3 \pm 1$ | $12 \pm 2$ | $17 \pm 3$ | $20 \pm 4$ | $8 \pm 2$ |

Differences are significant with control P<0,05
normalization of homeostasis parameters upon the above mentioned dose limits and method of usage of a declared preparation, controlled by the quantity of weight increases of calves, which declined from the norm in homeostasis parameters from the very beginning. Tables 9÷12 display the results of effectiveness of a declared preparation in case of:

- intramuscular injections with 3 day intervals (Table 9);
- inhalations with 24 hour intervals (Table 10);
- wet feedings with 7 day intervals (Table 11);
- intranasal administration with 12 hour intervals (Table 12).

The administration of a declared BAS in the form of a declared preparation affords a stimulation of:

- functional activity of liver tissue in hepatitis;
- regeneration of liver after hepatectomy;
- healing of ulcers, wounds, cutaneous surface in wounds and damages;
- reducing of blood formula in the cases of wounds and damages;
- treatment of inflammatory diseases (mastitis, pneumonias, endometritis);
- treatment of infectious diseases (parainfluenza, ARD);
- increase of the animal resistence to infectious and inflammatory processes;
- improvement of general biological state of the organism;
- rise of additional weight increase of animals.

The practical use of a declared BAS confirms its non-toxicity and absence of the negative side effects - allergy, delayed hypersensitivity.

The absence of side effects in a BAS used and a pharmacological preparation on its basis, and data of its pharmacological, therapeutical and general biological effects on animal organism determined the possibility of clinical approbation of a BAS and its preparations.

Clinical examination of patients receiving the declared BAS revealed positive influence of a preparation on physiological state of the organism, as well as practically full analogy with the preclinical experiments on animals.

The method of production of the declared BAS can be realized in the laboratory, and industrial conditions as well.

## EP 0 673 652 B1

THE SOURCES OF INFORMATION, TAKEN INTO CONSIDERATION

1. The request application of France No 2413912, A61K 35/44, 10.01.1978,
-Bontemps R.- The medicinal preparations based on an embryon and the method of their production.
2. The request application of E И ПВ No 0249563, A61K 35/54, 12.06.1987,
-Laumond Gerard. -The extracts of embryonal tissues of the animal organs, usable for human injections.
3. The patent of the USSR No 1442064, A61K 37/24, 4.10.1984.
- Robert Oertly (Solco Basel AG). -The mode of production of the extract with the immunomodulating effect, on the basis of animal raw material. (Rus.)
4. The authors copyright certificate of the USSR No 139404, A61K 35/12, 10.12.1960.
-N.G.Belenky and the others. -The method of production of the tissue preparations from animal raw material, such as spleen, placenta, embryons and so on, for the stimulation of the growth and weight increase of agricultural animals. (Rus.)
5. The patent of USA No 4455302, HK И 424-177, (A61K 37/00) 18.03.1982.
-H.J.Robertson.- Medical protein hydrolyzate, process of making the same and processes of utilizing the protein hydrolyzate to aid in healing traumatized areas.
6. The author certificate of the USSR No 904707, A61K 35/26, 35/28, 1982.
- A.M.Smirnov and others (Leningrad Veterinary Institute). The substance for increasing organism resistance of the younger animals and process for preparing. (Rus.)
7. Yasuo Kagava.Biomembranes.-M.-High -High school. -1985.-p. 13,15.
8. B.Alberts, D.Brey, J.Luis, The molecular biology of a cell. - "Mir"-1986.-p.97.
9. G.M. Higgins, R.M. Anderson. -Experimental pathology of the liver.- Arch. Pathology-1931-N12-p.186.
10. U.K. Laemmli. -Nature, -1970,-v. 227, -N5259, -p.680-685.
11. S.Seifter. "S.Dayton, C. Hovic. -Arch Biochemistry and Biophysics, -1950, -25, -1, -p.191-200.
12. J.M.Knight, S.Anderson, J.M.Rawle. - Clinical Chemistry -1972,-18,- p.199.
13. S.Reitman, S. Frankel. -American Journal of Clinical Pathology, -1957-28-56.
14. B.H.Brikker. The definition of K, Na contents by the method of flame photometry - Laboratornoye Delo,-1961,

**Claims**

1. A biologically active substance having immunomodulating properties, on the basis of organic compounds of the water-soluble hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue.

2. The substance as set forth in Claim 1, wherein said thermostable low-molecular mass components are present in an amount ranging from 30 to 100 percent by mass.

3. The substance as set forth in Claim 1, wherein said low-molecular mass components have a molecular mass ranging from 0.8 to 10.0 kDa.

4. The substance as set forth in Claim 1, wherein said organic compounds are present at the following ratio in percent by mass:

| | |
|---|---|
| Polypeptides | up to 26.0; |
| Peptides | from 10.0 to 22.5; |
| Amino acids | from 32.0 to 60.1; |
| Carbohydrates | from 10.5 to 28.7; |
| Lipids, nucleotides and other organic compounds | from 2.0 to 11.0. |

5. The substance as set forth in Claim 4, wherein carbohydrates are present in a composition of said organic compounds at the following ratio in percent by mass within:

| High-molecular mass compounds having a molecular mass of above 10.0 kDa | up to 2.0; |
|---|---|
| Low-molecular mass compounds having a molecular mass ranging from 0.8 to 10.0 kDa | from 2.5 to 11.5; |
| Free monomers having a molecular mass of less than 0.8 kDa | from 6.0 to 17.0. |

6. The substance as set forth in Claim 4, wherein said organic compounds are present at the following ratio in percent by mass:

| Polypeptides | from 16.3 to 24.7; |
|---|---|
| Peptides | from 11.9 to 17.1; |
| Amino acids | from 32.0 to 48.2; |
| Carbohydrates | from 14.6 to 20.8; |
| Lipids, nucleotides and other organic compounds | from 5.1 to 9.3. |

7. The substance as set forth in Claim 4, wherein said organic compounds are present at the following ratio in percent by mass:

| Polypeptides | from 5.5 to 12.7; |
|---|---|
| Peptides | from 13.6 to 21.0; |
| Amino acids | from 37.1 to 52.9; |
| Carbohydrates | from 16.1 to 25.1; |
| Lipids, nucleotides and other organic compounds | from 5.3 to 25.1. |

8. The substance as set forth in Claims 4, wherein said organic compounds are present at the following ratio in percent by mass:

| Polypeptides | less than 0.3; |
|---|---|
| Peptides | from 14.9 to 22.5; |
| Amino acids | from 43.5 to 60.1; |
| Carbohydrates | from 15.5 to 28.7; |
| Lipids, nucleotides and other organic compounds | from 5.0 to 9.2. |

9. The substance as set forth in any one of Claim 1 to 8, wherein a ratio between the mass content of carbohydrates and that of peptides, within said organic compounds having a molecular mass ranging from 0.8 to 10.0 kDa, ranges from 0.25 to 0.6.

10. A method for producing a biologically active substance having immunomodulating properties, on the basis of organic compounds of the hydrolysis products of disintegrated animal tissue to the exclusion of tissue from human embryos, comprising components, which are thermostable at a temperature of μp to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue, comprising disintegrating the washed raw material of animal tissue with subsequent homogenization in physiological and/or buffer solutions, separating non-disintegrated tissue elements by means of centrifugation at 150 to 250 x g for 10 to 15 minutes and/or filtration, isolating supernatant, hydrolyzing said supernatant, separating the hydrolysis products by means of centrifugation at above 1 500 X g for 20 to 40 minutes and/or filtration, and collecting of the water-soluble hydrolysis products as desired product.

11. The method as set forth in Claim 10, wherein said raw material is selected from the group comprising embryonic tissue to the exclusion of tissue from human embryos, differentiating thymocytes of the thymus, spermatogones and spermatocytes of the testes, placental tissue, epithelial tissue of the intestine, bone marrow cells, regenerating cutaneous tissue, regenerating hepatic tissue, tissue of regenerating amphibian limbs, pathologically transformed tissue taken prior to the stage of regeneration, and any combination thereof.

12. The method as set forth in Claim 10, wherein said disintegrating of raw material, prior to homogenization, is adjusted up to damaging integrity of the cells, with subsequent extraction of their content by means of an additional washing with filtration and/or centrifugation at 300 to 15 000 x g for 10 to 20 minutes, followed by separating extracted disintegrated tissue.

13. The method as set forth in Claim 10, wherein said supernatant isolated following separating non-disintegrated tissue elements contains components of morphoplasm and glycocalyx of the cells in the composition of organic compounds from 30 to 60 percent by mass.

14. The method as set forth in Claim 10, wherein said hydrolysis is performed at a temperature ranging from 4°C to 45°C.

15. The method as set forth in Claim 10, wherein said hydrolysis is performed in the presence of a preservative.

16. The method as set forth in any one of Claims 10 to 15, wherein said hydrolysis products are subjected to thermal treatment at a temperature ranging from 60°C to 120°C up to complete denaturation of thermolabile compounds by means of filtration and/or centrifugation at above I 500 x g for 20 to 40 minutes with collecting supernatant containing thermostable components as desired product.

17. The method as set forth in any one of Claims 10 to 15, wherein said hydrolysis products are subjected to fractionation by means of dialysis through a semipermeable membrane and/or ultrafiltration and/or gelfiltration with collecting a fraction of low-molecular mass components having a molecular mass less than 10.0 kDa as desired product.

18. The method as set forth in Claim 16, wherein said thermostable components of hydrolysis products are subjected to fractionation by means of dialysis through a semipermeable membrane and/or ultrafiltration and/or gelfiltration with collecting a fraction of low-molecular mass components having a molecular mass less than 10.0 kDa as desired product.

19. A preparation for the normalization of the physiological state of human and animal organisms, consisting of a biologically active substance having immunomodulating properties on the basis of organic compounds of the water-soluble hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue, said substance being taken in an amount ranging from 0.001 to 85.0 percent by mass with the rest being an excipient.

20. The preparation as set forth in Claim 19, wherein said biologically active substance comprises organic compounds at the following ratio in percent by mass:

| Polypeptides | up to 26.0; |
|---|---|
| Peptides | from 10.0 to 22.5; |
| Amino acids | from 32.0 to 60.1; |
| Carbohydrates | from 10.5 to 28.7; |
| Lipids, nucleotides and other organic compounds | from 2.0 to 11.0. |

21. The preparation as set forth in Claim 19, wherein said biologically active substance comprises organic compounds at the following ratio in percent by mass:

| Polypeptides | from 16.3 to 24.7; |
|---|---|
| Peptides | from 11.9 to 17.1; |
| Amino acids | from 32.0 to 48.2; |
| Carbohydrates | from 14.6 to 20.8; |
| Lipids, nucleotides and other organic compounds | from 5.1 to 9.3. |

22. The preparation as set forth in Claim 19, wherein said biologically active substance comprises organic compounds at the following ratio in percent by mass:

| Polypeptides | from 5.5 to 12.7; |
|---|---|
| Peptides | from 13.6 to 21.0; |
| Amino acids | from 37.1 to 52.9; |
| Carbohydrates | from 16.1 to 25.1; |
| Lipids, nucleotides and other organic compounds | from 5.3 to 11.0. |

23. The preparation as set forth in Claim 19, wherein said biologically active substance comprises organic compounds at the following ratio in percent by mass:

| Polypeptides | less than 0.3; |
|---|---|
| Peptides | from 14.9 to 22.5; |
| Amino acids | from 43.5 to 60.1; |
| Carbohydrates | from 15.5 to 28.7; |
| Lipids, nucleotides and other organic compounds | from 5.0 to 9.2. |

24. The preparation as set forth in any one of Claims 19 to 23, wherein an excipient is present as physiological solution of sodium chloride and/or saline buffer solution and/or carbohydrate compounds and/or fat of minerals, animals, vegetables or synthetic origin or any mixture thereof.

25. The preparation as set forth in any one of Claims 19 to 23, wherein a preservative is present additionally.

26. Use of a biologically active substance having immunomodulating properties on the basis of organic compounds of the water-soluble hydrolysis products of disintegrated animal tissue to the exclusion of tissue from human embryos, comprising components, which are thermostable at a temperature of up to 120°C with low-molecular mass less than 10.0 kDa isolated from morphoplasm and glycocalyx of the cells, taken from the processes of embryogenesis and/or proliferation and/or differentiation of the cells, and/or pathology preceding and/or accompanying the process of regeneration and/or reparation of the tissue, for the manufacture of a preparation for the normalization of the physiological state of human and animal organisms, said preparation being suitable for administration to said organisms and consisting of said biologically active substance in an amount ranging from 0.001 to 85.0 percent by mass, with the rest being an excipient.

27. The use as set forth in Claim 26, wherein said preparation is administered as intramuscular injections at a dose ranging from 0.005 to 0.5 mg/kg of the body weight with 1 to 7 day intervals between injections and/or inhalations at a dose ranging from 0.012 to 0.12 mg/kg of the body weight with 4 to 48 hour intervals between inhalations and/or sublingual administrations at a dose ranging from 0.012 to 0.12 mg/kg of the body weight with 4 to 48 hour intervals between administrations and/or wet feedings at a dose ranging from 0.02 to 1 mg/kg of the body weight with 1 to I0 day intervals between feedings and/or oral pills at a dose ranging from 0.02 to 1 mg/kg of the body weight with 1 to I0 day intervals between administrations and/or intranasal administrations at a daily dose ranging from 0.001 to 0.05 mg/kg of the body weight with 2 to 24 hour intervals between administrations till the normalization of physiological parameters.

28. The use as set forth in Claim 26, wherein said preparation is administered as applications to mucous or wound surfaces in the form of compresses, suppositories, ointments, powders and gels with 1 to 10 day intervals between applications till the normalization of physiological parameters.

**Patentansprüche**

1. Biologisch wirksamer Stoff mit immunmodulierenden Eigenschaften auf der Basis organischer Verbindungen wasserlöslicher Hydrolyseprodukte zersetzter tierischer Gewebe unter Ausschluß von Geweben aus menschlichen Embryonen, wobei dieser Stoff Komponenten umfaßt, die bei einer Temperatur von bis zu 120°C bei einer niedermolekularen Masse von unter 10.0 kDa wärmebeständig sind und aus dem Morphoplasma und der Glyco- calyx von Zellen isoliert sind, die aus Prozessen der Embryogenese und/oder Vermehrung und/oder Differenzierung der Zellen und/oder der Pathologie, welche dem Prozeß der Regeneration und/ oder der Reparatur des Gewebes vorausgeht und/oder diesen begleitet, stammen.

2. Stoff nach Anspruch 1, bei dem die wärmebeständigen niedermolekularen Massekomponenten in einer Menge von 30 bis 100 Masseprozent vorliegen.

3. Stoff nach Anspruch 1, bei dem die niedermolekularen Massekomponenten eine Molekularmasse von 0.8 bis 10,0 kDa besitzen.

4. Stoff nach Anspruch 1, bei dem die organischen Verbindungen bei folgendem Verhältnis in Masseprozent vorliegen:

| | |
|---|---|
| Polypeptide | bis 26,0 |
| Peptide | 10,0 - 22,5 |
| Aminosäuren | 32,0 - 60,1 |
| Kohlenhydrate | 10,5 - 28,7 |
| Lipide, Nucleotide und andere organische Verbindungen | 2,0 - 11,0. |

5. Stoff nach Anspruch 4, bei dem die Kohlenhydrate in einem Gemisch der organischen Verbindungen bei folgendem Verhältnis in Masseprozent vorliegen:

| | |
|---|---|
| Höhermolekulare Verbindungen mit einer Molekularmasse von über 10,0 kDa | bis 2,0 |
| Niedermolekulare Verbindungen mit einer Molekularmasse von 0,8 bis 10,0 kDa | 2,5 - 11,5 |
| Freie Monomere mit einer Molekularmasse -von unter 0,8 kDa | 6,0 - 17,0. |

6. Stoff nach Anspruch 4, bei dem die organischen Verbindungen bei folgendem Verhältnis in Masseprozent vorliegen:

| | |
|---|---|
| Polypeptide | 16,3 - 24,7 |
| Peptide | 11,9 - 17,1 |
| Aminosäuren | 32,0 - 48,2 |
| Kohlenhydrate | 14,6 - 20,8 |
| Lipide, Nucleotide und andere organische Verbindungen | 5,1 - 9,3. |

7. Stoff nach Anspruch 4, bei dem die organischen Verbindungen bei folgendem Verhältnis in Masseprozent vorliegen:

| | |
|---|---|
| Polypeptide | 5,5 - 12,7 |
| Peptide | 13,6 - 21,0 |
| Aminosäuren | 37,1 - 52,9 |
| Kohlenhydrate | 16,1 - 25,1 |
| Lipide, Nucleotide und andere organische Verbindungen | 5,3 - 25,1. |

8. Stoff nach Anspruch 4, bei dem die organischen Verbindungen bei folgendem Verhältnis in Masseprozent vorliegen:

| | |
|---|---|
| Polypeptide | unter 0,3 |
| Peptide | 14,9 - 22,5 |
| Aminosäuren | 43,5 - 60,1 |
| Kohlenhydrate | 15,5 - 28,7 |
| Lipide, Nucleotide und andere organische Verbindungen | 5,0 - 9,2. |

9. Stoff nach einem der Ansprüche 1 - 8 bei einem Verhältnis zwischen dem Massegehalt an Kohlenhydraten und dem an Peptiden von 0,25 - 0,6, wobei die organischen Verbindungen eine Molekularmasse von 0,8 - 10,0 kDa aufweisen.

10. Verfahren zur Herstellung eines biologisch wirksamen Stoffes mit immunmodulierenden Eigenschaften auf der Basis organischer Verbindungen der Hydrolyseprodukte zersetzter tierischer Gewebe unter Ausschluß von Geweben aus menschlichen Embryonen, wobei dieser Stoff Komponenten umfaßt, die bei einer Temperatur von bis zu 120°C bei einer niedermolekularen Masse von unter 10.0 kDa wärmebeständig sind und aus dem Morphoplasma und der Glycocalyx von Zellen isoliert sind, die aus Prozessen der Embryogenese und/oder Vermehrung und/oder Differenzierung der Zellen und/oder der Pathologie, welche dem Prozeß der Regeneration und/oder der Reparatur des Gewebes vorausgeht und/oder diesen begleitet, stammen, das die Zersetzung des gewaschenen Ausgangsmaterials aus tierischem Gewebe unter nachfolgender Homogenisierung in physiologischer Kochsalzlösung und/oder einer Pufferlösung, die Abtrennung der nichtzersetzten Gewebeelemente durch Zentrifugieren bei 150 - 250 x g während 10- 15 min und/oder das Filtrieren, Isolieren des Überstandes, die Hydrolyse des Überstandes, die Abtrennung der Hydrolyseprodukte durch Zentrifugieren bei 1500 x g während 20 - 40 min und/oder das Filtrieren und das Sammeln der wasserlöslichen Hydrolyseprodukte als erwünschtes Produkt umfaßt.

11. Verfahren nach Anspruch 10, bei dem der Ausgangsstoff ausgewählt wird aus der Gruppe, die embryonales Gewebe unter Ausschluß von Geweben aus menschlichen Embryonen, differenzierende Thymocyten der Thymusdrüse, Spermatogonien und Spermatocyten der Hoden, Plazentagewebe, Epithelgewebe des Darms, Knochenmarkzellen, regenerierendes Hautgewebe, regenerierendes Lebergewebe, Gewebe von regenerierenden Amphibienextremitäten, pathologisch verändertes Gewebe, das vor dem Stadium der Regeneration entnommen wurde, und jede Kombination davon umfaßt.

12. Verfahren nach Anspruch 10, bei dem die Zersetzung des Ausgangsstoffes vor der Homogenisierung so eingestellt wird, daß die Integrität der Zellen geschädigt wird, wonach ihr Inhalt durch zusätzliches Waschen unter Filtrieren und/oder Zentrifugieren bei 300 bis 15.000 x g während 10 bis 20 min extrahiert wird, worauf das extrahierte zersetzte Gewebe abgetrennt wird.

13. Verfahren nach Anspruch 10, bei dem der isolierte Überstand nach Abtrennen der nichtzersetzten Gewebeelemente Komponenten des Morphoplasmas und der Glycocalyx der Zellen im Gemisch aus organischen Verbindungen von 30 - 60 Masseprozent enthält.

14. Verfahren nach Anspruch 10, bei dem die Hydrolyse bei einer Temperatur von 4 - 45°C durchgeführt wird.

15. Verfahren nach Anspruch 10, bei dem die Hydrolyse in Anwesenheit eines Konservierungsmittels durchgeführt wird.

16. Verfahren nach einem der Ansprüche 10 - 15, bei dem die Hydrolyseprodukte einer Wärmebehandlung bei einer Temperatur von 60 - 120°C bis zur vollständigen Denaturierung der wärmeunbeständigen Verbindungen durch Filtrieren und/oder Zentrifugieren bei über 1500 x g während 20 - 40 min unterworfen werden, wobei der die wärmebeständigen Komponenten als erwünschtes Produkt enthaltende Überstand gesammelt wird.

17. Verfahren nach einem der Ansprüche 10 - 15, bei dem die Hydrolyseprodukte durch Dialyse durch eine semipermeable Membran und/oder Ultrafiltration und/oder Gelfiltration fraktioniert werden, wonach eine Fraktion aus niedermolekularen Komponenten mit einer Molekularmasse von unter 10.0 kDa als erwünschtes Produkt gesammelt wird.

18. Verfahren nach Anspruch 16, bei dem die wärmebeständigen Komponenten der Hydrolyseprodukte durch Dialyse durch eine semipermeable Membran und/oder Ultrafiltration und/oder Gelfiltration fraktioniert werden, wonach eine Fraktion aus niedermolekularen Komponenten mit einer Molekularmasse von unter 10.0 kDa als erwünschtes Produkt gesammelt wird.

19. Präparat zur Normalisierung des physiologischen Zustandes des menschlichen und tierischen Organismus, das aus einem biologisch wirksamen Stoff mit immunmodulierenden Eigenschaften auf der Basis organischer Verbindungen wasserlöslicher Hydrolyseprodukte zersetzter tierischer Gewebe unter Ausschluß von Geweben aus menschlichen Embryonen besteht, wobei dieser Stoff Komponenten umfaßt, die bei einer Temperatur von bis zu 120°C bei einer niedermolekularen Masse von unter 10.0 kDa wärmebeständig sind, und aus dem Morphoplasma und der Glycocalyx von Zellen isoliert sind, die aus Prozessen der Embryogenese und/oder Vermehrung und/oder Differenzierung der Zellen und/oder der Pathologie, welche dem Prozeß der Regeneration und/oder der Reparatur des Gewebes vorausgeht und/oder diesen begleitet, stammen, und dieser Stoff in einer Menge von 0.001 - 85.0 Masseprozent eingesetzt wird, wobei der Rest auf einen Träger entfällt.

20. Präparat nach Anspruch 19, bei dem der biologisch wirksame Stoff organische Verbindungen bei folgendem Verhältnis in Masseprozent umfaßt:

| | |
|---|---|
| Polypeptide | bis 26,0 |
| Peptide | 10,0 - 22,5 |
| Aminosäuren | 32,0 - 60,1 |
| Kohlenhydrate | 10,5 - 28,7 |
| Lipide, Nucleotide und andere organische Verbindungen | 2,0 - 11,0. |

21. Präparat nach Anspruch 19, bei dem der biologisch wirksame Stoff organische Verbindungen bei folgendem Verhältnis in Masseprozent umfaßt:

| | |
|---|---|
| Polypeptide | 16,3 - 24,7 |
| Peptide | 11,9 - 17,1 |
| Aminosäuren | 32,0 - 48,2 |
| Kohlenhydrate | 14,6 - 20,8 |
| Lipide, Nucleotide und andere organische Verbindungen | 5,1 - 9,3. |

22. Präparat nach Anspruch 19, bei dem der biologisch wirksame Stoff organische Verbindungen bei folgendem Verhältnis in Masseprozent umfaßt:

| | |
|---|---|
| Polypeptide | 5,5 - 12,7 |
| Peptide | 13,6 - 21,0 |
| Aminosäuren | 37,1 - 52,9 |
| Kohlenhydrate | 16,1 - 25,1 |
| Lipide, Nucleotide und andere organische Verbindungen | 5,3 - 25,1. |

23. Präparat nach Anspruch 19, bei dem der biologisch wirksame Stoff organische Verbindungen bei folgendem Verhältnis in Masseprozent umfaßt:

| | |
|---|---|
| Polypeptide | unter 0,3 |
| Peptide | 14,9 - 22,5 |
| Aminosäuren | 43,5 - 60,1 |
| Kohlenhydrate | 15,5 - 28,7 |
| Lipide, Nucleotide und andere organische Verbindungen | 5,0 - 9,2. |

24. Präparat nach einem der Ansprüche 19 - 23, bei dem ein Träger als physiologische Lösung- aus Natriumchlorid und/oder als Kochsalzpufferlösung und/oder Kohlenhydratverbindungen und/ oder Fette von Mineralien, Tieren, Pflanzen oder synthetischer Herkunft oder als Gemisch davon vorliegt.

25. Präparat nach einem der Ansprüche 19 -23, bei dem zusätzlich noch ein Konservierungsmittel vorliegt.

26. Verwendung eines biologisch wirksamen Stoffes mit immunmodulierenden Eigenschaften auf der Basis organischer Verbindungen wasserlöslicher Hydrolyseprodukte zersetzter tierischer Gewebe unter Ausschluß von Geweben aus menschlichen Embryonen, wobei dieser Stoff Komponenten umfaßt, die bei einer Temperatur von bis zu 120°C bei einer niedermolekularen Masse von unter 10.0 kDa wärmebeständig sind und aus dem Morphoplasma und der Glycocalyx von Zellen isoliert sind, die aus Prozessen der Embryogenese und/oder Vermehrung und/ oder Differenzierung der Zellen und/oder der Pathologie, welche dem Prozeß der Regeneration und/oder der Reparatur des Gewebes vorausgeht und/oder diesen begleitet, stammen, zur Herstellung eines Präparats für die Normalisie rung des physiologischen Zustandes des menschlichen oder tierischen Organismus, wobei es für die Verabreichung an Organismen geeignet ist und aus dem biologisch wirksamen Stoff in einer Menge von 0.001 - 85.0 Masseprozent besteht, wobei der Rest auf einen Träger entfällt.

**27.** Verwendung nach Anspruch 26, wobei das Präparat in Form intramuskulärer Injektionen bei einer Dosierung von 0.005 - 0.5 mg/kg des Körpergewichts bei Intervallen von 1 - 7 Tagen zwischen den einzelnen Injektionen und/ oder in Form von Inhalationen bei einer Dosierung von 0.012 - 0.12 mg/kg des Körpergewichts bei Intervallen von 4 - 48 h zwischen den einzelnen Inhalationen und/oder in Form sublingualer Verabreichungen bei einer Dosierung von 0.012 - 0.12 mg/kg des Körpergewichts bei Intervallen von 4 - 48 zwischen den einzelnen Verabreichungen und/oder in flüssiger Form bei einer Dosierung von 0.02 - 1.0 mg/kg des Körpergewichts bei Intervallen von 1 - 10 Tagen zwischen den einzelnen Verabreichungen und/oder in Form oral zu verabreichender Pillen bei einer Dosierung von 0.02 - 1 mg/kg des Körpergewichts bei Intervallen von 1 - 10 Tagen zwischen den einzelnen Verabreichungen und/oder in Form intranasaler Verabreichungen bei einer Tagesdosis von 0.001 - 0.05 mg/kg des Körpergewichts bei Intervallen von 2 - 24 h zwischen den einzelnen Verabreichungen bis zur Normalisierung der physiologischen Parameter verabreicht wird.

**28.** Verwendung nach Anspruch 26, bei dem das Präparat auf Schleimhäute oder Wμndoberflächen in Form von Kompressen, Suppositorien, Salben, Pulvern und Gelen bei Intervallen von 1 - 10 Tagen zwischen den einzelnen Verabreichungen bis zur Normalisierung der physiologischen Parameter verabreicht wird.


**Revendications**

**1.** Substance biologiquement active ayant des propriétés d'immunomodulation, à base de composés organiques des produits d'hydrolyse hydrosolubles d'un tissu animal désintégré à l'exclusion d'un tissu provenant d'embryons humains, comprenant des composants qui sont thermostables à une température allant jusqu'à 120°C avec une faible masse moléculaire inférieure à 10,0 kDa isolés à partir du morphoplasme et du glycocalyx des cellules, pris durant les processus d'embryogenèse et/ou de prolifération et/ou de différenciation des cellules, d'une pathologie précédant et/ou accompagnant le processus de régénération et/ou de réparation du tissu.

**2.** Substance selon la revendication 1, dans laquelle lesdits composants thermostables de faible masse moléculaire sont présents en une quantité variant de 30 à 100% en masse.

**3.** Substance selon la revendication 1, dans laquelle lesdits composants de faible masse moléculaire ont une masse moléculaire variant de 0,8 à 10,0 kDa.

**4.** Substance selon la revendication 1, dans laquelle lesdits composés organiques sont présents dans le rapport suivant, en pourcentage en masse :

| | |
|---|---|
| Polypeptides | jusqu'à 26,0 ; |
| Peptides | de 10,0 à 22,5 ; |
| Acides aminés | de 32,0 à 60,1 ; |
| Glucides | de 10,5 à 28,7 ; |
| Lipides, nucléotides et autres composés organiques | de 2,0 à 11,0. |

**5.** Substance selon la revendication 4, dans laquelle les glucides sont présents dans une composition desdits composés organiques dans le rapport suivant, en pourcentage en masse :

| | |
|---|---|
| Composés de masse moléculaire élevée ayant une masse moléculaire supérieure à 10,0 kDa | jusqu'à 2,0 ; |
| Composés de faible masse moléculaire ayant une masse moléculaire variant de 0,8 à 10,0 kDa | de 2,5 à 11,5 ; |
| Monomères libres ayant une masse moléculaire inférieure à 0,8 kDa | de 6,0 à 17,0 ; |

**6.** Substance selon la revendication 4, dans laquelle lesdits composés organiques sont présents dans le rapport suivant, en pourcentage en masse :

| | |
|---|---|
| Polypeptides | de 16,3 à 24,7 ; |
| Peptides | de 11,9 à 17,1 ; |
| Acides aminés | de 32,0 à 48,2 ; |
| Glucides | de 14,6 à 20,8 ; |
| Lipides, nucléotides et autres composés organiques | de 5,1 à 9,3. |

**7.** Substance selon la revendication 4, dans laquelle lesdits composés organiques sont présents dans le rapport suivant, en pourcentage en masse :

| Polypeptides | de 5,5 à 12,7 ; |
|---|---|
| Peptides | de 13,6 à 21,0; |
| Acides aminés | de 37,1 à 52,9 ; |
| Glucides | de 16,1 à 25,1 ; |
| Lipides, nucléotides et autres composés organiques | de 5,3 à 25,1. |

**8.** Substance selon la revendication 4, dans laquelle lesdits composés organiques sont présents dans le rapport suivant, en pourcentage en masse :

| Polypeptides | moins de 0,3 ; |
|---|---|
| Peptides | de 14,9 à 22,5; |
| Acides aminés | de 43,5 à 60,1 ; |
| Glucides | de 15,5 à 28,7; |
| Lipides, nucléotides et autres composés organiques | de 5,0 à 9,2. |

**9.** Substance selon l'une quelconque des revendications 1 à 8, dans laquelle le rapport entre la teneur massique des glucides et celle des peptides, dans lesdits composés organiques ayant une masse moléculaire variant de 0,8 à 10,0 kDa, va de 0,25 à 0,6.

**10.** Procédé de production d'une substance biologiquement active ayant des propriétés d'immunomodulation, à base de composés organiques des produits d'hydrolyse d'un tissu animal désintégré à l'exclusion d'un tissu provenant d'embryons humains, comprenant des composants qui sont thermostables à une température allant jusqu'à 120°C avec une faible masse moléculaire inférieure à 10,0 kDa isolés à partir du morphoplasme et du glycocalyx des cellules, pris durant les processus d'embryogenèse et/ou de prolifération et/ou de différenciation des cellules, et/ou d'une pathologie précédant et/ou accompagnant le processus de régénération et/ou de réparation du tissu, comprenant la désintégration de la matière première lavée de tissu animal avec une homogénéisation ultérieure dans des solutions tampons et/ou physiologiques, la séparation des éléments tissulaires non désintégrés au moyen d'une centrifugation à 150-250 x g pendant 10 à 15 minutes et/ou la filtration ou l'isolement du surnageant, l'hydrolyse dudit surnageant, la séparation des produits d'hydrolyse au moyen d'une centrifugation au dessus de 1500 x g pendant 20 à 40 minutes et/ou la filtration, et la récupération des produits d'hydrolyse hydrosolubles représentant le produit désiré.

**11.** Procédé selon la revendication 10, dans lequel ladite matière première est choisie dans le groupe comprenant du tissu embryonnaire à l'exclusion du tissu d'embryons humains, des thymocytes en voie de différenciation du thymus, des spermatogonies et des spermatocytes des testicules, du tissu placentaire, du tissu épithélial de l'intestin, des cellules de moelle osseuse, du tissu cutané régénérescent, du tissu hépatique régénérescent, du tissu de membres d'amphibien en voie de régénération, du tissu pathologiquement transformé pris avant le stade de régénération, et toute combinaison des précédents.

**12.** Procédé selon la revendication 10, dans lequel ladite désintégration de matière première, avant homogénéisation, est mise au point de manière à endommager l'intégrité des cellules, avec une extraction ultérieure de leur contenu au moyen d'un lavage supplémentaire avec filtration et/ou centrifugation à 300-15 000 x g pendant 10 à 20 minutes, suivie par une séparation du tissu désintégré extrait.

**13.** Procédé selon la revendication 10, dans lequel ledit surnageant isolé à la suite de la séparation des éléments tissulaires non désintégrés contient des composants de morphoplasme et de glycocalyx des cellules dans la composition de composés organiques à raison de 30 à 60% en masse.

**14.** Procédé selon la revendication 10, dans lequel ladite hydrolyse est effectuée à une température variant de 4°C à 45°C.

**15.** Procédé selon la revendication 10, dans lequel ladite hydrolyse est effectuée en présence d'un conservateur.

**16.** Procédé selon l'une quelconque des revendications 1 à 15, dans lequel lesdits produits d'hydrolyse sont soumis à un traitement thermique à une température variant de 60°C à 120°C jusqu'à dénaturation complète des composés thermolabiles au moyen d'une filtration et/ou centrifugation au dessus de 1500 x g pendant 20 à 40 minutes avec récupération du surnageant contenant des composants thermostables représentant le produit désiré.

**17.** Procédé selon l'une quelconque des revendications 10 à 15, dans lequel lesdits produits d'hydrolyse sont soumis à un fractionnement au moyen d'une dialyse à travers une membrane servi-perméable et/ou d'une ultrafiltration et/ou d'une filtration sur gel avec récupération d'une fraction de composants de faible masse moléculaire ayant une masse moléculaire inférieure à 10,0 kDa représentant le produit désiré.

**18.** Procédé selon la revendications 16, dans lequel lesdits composants thermostables des produits d'hydrolyse sont soumis à un fractionnement au moyen d'une dialyse à travers une membrane semi-perméable et/ou d'une ultrafiltration et/ou d'une filtration sur gel avec récupération d'une fraction de composants de faible masse moléculaire ayant une masse moléculaire inférieure à 10,0 kDa représentant le produit désiré.

**19.** Préparation destinée à la normalisation de l'état physiologique d'organismes humains et animaux, consistant en une substance biologiquement active ayant des propriétés d'immunomodulation à base de composés organiques des produits d'hydrolyse hydrosolubles d'un tissu animal désintégré à l'exclusion d'un tissu provenant d'embryons humains, comprenant des composants qui sont thermostables à une température allant jusqu'à 120°C avec une faible masse moléculaire inférieure à 10,0 kDa isolés à partir du morphoplasme et du glycocalyx des cellules, pris durant les processus d'embryogenèse et/ou de prolifération et/ou de différenciation des cellules, d'une pathologie précédant et/ou accompagnant le processus de régénération et/ou de réparation du tissu, ladite substance étant prise en une quantité variant de 0,001 à 85,0% en masse, le reste étant un excipient.

**20.** Préparation selon la revendication 19, dans laquelle ladite substance biologiquement active comprend des composés organiques dans le rapport suivant, en pourcentage en masse :

| | |
|---|---|
| Polypeptides | jusqu'à 26,0 ; |
| Peptides | de 10,0 à 22,5; |
| Acides aminés | de 32,0 à 60,1 ; |
| Glucides | de 10,5 à 28,7 ; |
| Lipides, nucléotides et autres composés organiques | de 2,0 à 11,0. |

**21.** Préparation selon la revendication 19, dans laquelle ladite substance biologiquement active comprend des composés organiques dans le rapport suivant, en pourcentage en masse :

| | |
|---|---|
| Polypeptides | de 16,3 à 24,7 ; |
| Peptides | de 11,9 à 17,1 ; |
| Acides aminés | de 32,0 à 48,2 ; |
| Glucides | de 14,6 à 20,8 ; |
| Lipides, nucléotides et autres composés organiques | de 5,1 à 9,3. |

**22.** Préparation selon la revendication 19, dans laquelle ladite substance biologiquement active comprend des composés organiques dans le rapport suivant, en pourcentage en masse :

| | |
|---|---|
| Polypeptides | de 5,5 à 12,7 ; |
| Peptides | de 13,6 à 21,0; |
| Acides aminés | de 37,1 à 52,9 ; |
| Glucides | de 16,1 à 25,1 ; |
| Lipides, nucléotides et autres composés organiques | de 5,3 à 11,0. |

**23.** Préparation selon la revendication 19, dans laquelle ladite substance biologiquement active comprend des composés organiques dans le rapport suivant, en pourcentage en masse :

| | |
|---|---|
| Polypeptides | moins de 0,3 ; |

(suite)

| Peptides | de 14,9 à 22,5; |
|----------|-----------------|
| Acides aminés | de 43,5 à 60,1 ; |
| Glucides | de 15,5 à 28,7; |
| Lipides, nucléotides et autres composés organiques | de 5,0 à 9,2. |

24. Préparation selon l'une quelconque des revendications 19 à 23, dans laquelle un excipient est présent sous forme de solution physiologique de chlorure de sodium et/ou de solution tampon saline et/ou de composés glucidiques et/ou de graisse d'origine minérale, animale, végétale ou synthétique ou tout mélange des précédents.

25. Préparation selon l'une quelconque des revendications 19 à 23, dans laquelle un conservateur est présent en plus.

26. Utilisation d'une substance biologiquement active ayant des propriétés d'immunomodulation à base de composés organiques des produits d'hydrolyse hydrosolubles d'un tissu animal désintégré à l'exclusion d'un tissu provenant d'embryons humains, comprenant des composants qui sont thermostables à une température allant jusqu'à 120°C avec une faible masse moléculaire inférieure à 10,0 kDa isolés à partir du morphoplasme et du glycocalyx des cellules, pris durant les processus d'embryogenèse et/ou de prolifération et/ou de différenciation des cellules, et/ou d'une pathologie précédant et/ou accompagnant le processus de régénération et/ou de réparation du tissu, pour la fabrication d'une préparation destinée à la normalisation de l'état physiologique d'organismes humains et animaux, ladite préparation convenant à l'administration auxdits organismes et consistant en ladite substance biologiquement active en une quantité variant de 0,001 à 85,0% en masse, le reste étant un excipient.

27. Utilisation selon la revendication 26, dans laquelle ladite préparation est administrée sous forme d'injections intramusculaires à une dose variant de 0,005 à 0,5 mg/kg du poids corporel avec des intervalles de 1 à 7 jours entre les injections et/ou sous forme d'inhalations à une dose variant de 0,012 à 0,12 mg/kg du poids corporel avec des intervalles de 4 à 48 heures entre les inhalations et/ou sous forme d'administrations sublinguales à une dose variant de 0,012 à 0,12 mg/kg du poids corporel avec des intervalles de 4 à 48 heures entre les administrations et/ou sous forme d'aliments humides à une dose variant de 0,02 à 1,0 mg/kg du poids corporel avec des intervalles de 1 à 10 jours entre les aliments et/ou sous forme de pilules orales à une dose variant de 0,02 à 1,0 mg/kg du poids corporel avec des intervalles de 1 à 10 jours entre les administrations et/ou sous forme d'administrations intranasales à une dose journalière variant de 0,001 à 0,05 mg/kg du poids corporel avec des intervalles de 2 à 24 heures entre les administrations jusqu'à la normalisation des paramètres physiologiques.

28. Utilisation selon la revendication 26, dans laquelle ladite préparation est administrée en applications sur des surfaces de muqueuses ou de plaies sous forme de compresses, de suppositoires, d'onguents, de poudres et de gels avec des intervalles de 1 à 10 jours entre les applications jusqu'à la normalisation des paramètres physiologiques.